# EUROPEAN PATENT APPLICATION

(11) **EP 4 678 637 A1**
(43) Date of publication of application: **14.01.2026**
(21) Application number: 24767462.5
(22) Date of filing: 11.03.2024
(51) Int. Cl.: C07D 311/26, A61K 31/352, A61P 43/00, A61P 11/00, A23L 33/10, C07D 311/36

(54) **NOVEL FLAVONE DERIVATIVE AND USE THEREOF IN ALLEVIATING PULMONARY FIBROSIS**

(30) Priority: 09.03.2023 KR 20230031226; 27.06.2023 KR 20230082360
(71) Applicant: A-chembio Co., Ltd, Jeju-si, Jeju 63169 (KR); Jeju National University Industry-Academic Cooperation Foundation, Jeju-si, Jeju-do 63243 (KR)
(72) Inventor: CHO, Moon Jae, Jeju-si Jeju-do 63240 (KR); KIM, Young Mee, Jeju-si Jeju-do 63182 (KR); KIM, Sang Jun, Anyang-si Gyeonggi-do 14106 (KR)
(74) Representative: V.O.
(86) International application number: PCT/KR2024/003103
(87) International publication number: WO 2024/186175

(57) **Abstract**

Proposed are a novel flavone derivative and a composition for improving pulmonary fibrosis using the same. The flavone derivative inhibits the TGF-β1 signaling pathway without showing specific cytotoxicity to A549 cells, derived from lung cancer, Hulec-5a cells, derived from pulmonary vessels, and human lung fibroblasts (HLF), thereby inhibiting epithelial-mesenchymal transition (EMT). Additionally, the flavone derivative also shows the effect of regulating the expression of pulmonary fibrosis-related factors in the A549 cells and Hulec-5a cells treated with epidermal growth factor (EGF) or bleomycin, which causes pulmonary fibrosis.

## Description

### Technical Field

The present disclosure relates to a novel flavone derivative and a use thereof for improving pulmonary fibrosis.

### Background Art

Pulmonary fibrosis or idiopathic pulmonary fibrosis (IPF) is a disease characterized by fibrosis of the lung interstitium in which connective tissue, particularly collagen, is excessively engrafted in the alveolar wall. Pulmonary fibrosis is a fatal disease that progresses to respiratory failure due to a gradual decline in lung function and has an average survival period of less than 2-3 years after diagnosis. The prevalence of pulmonary fibrosis varies from report to report, but it is known to be 2-29 out of 100,000 people and is designated as a rare and intractable disease in Korea.

Pulmonary fibrosis is a disease that occurs in the elderly and is expected to increase gradually as the population ages. Recent research results have reported that the prevalence of pulmonary fibrosis reported to date is underestimated, reporting that about 7-8% of smokers or the general population. Therefore, it is expected that a considerable number of older people (currently, 1 in 450 men over 70 years of age) will suffer from this disease, but there is still no effective treatment method. Lung transplants also require a long waiting time because the number of transplant donors is smaller than the number of patients who need to receive a transplant (so many of the patients come to death while waiting for a transplant because the disease continuously worsens). Even if the patients fortunately receive a lung transplant, many of them fail to survive due to complications after the transplant (5-year survival rate is about 50%). Therefore, a more effective and safe treatment method is urgently needed.

The causes of pulmonary fibrosis are diverse, including lung damage, exposure to toxic substances or toxic environments such as fine dust or ultrafine dust, anticancer drugs, autoimmune diseases, or idiopathic interstitial pneumonia (Proc Am Thorac Soc 2006, 3:285-92; Am J Respir Crit Care Med 2002, 165:277-304). The basic histological changes in pulmonary fibrosis include inflammatory changes such as cell infiltration, edema, and exudation in the alveolar septum and the destruction of lung parenchymal by infiltration and fibrosis of extracellular matrixes such as collagen, proteoglycan, fibronectin, and glycoprotein (Am J Respir Crit Care Med 2002, 165:277-304; N Engl J Med 2001, 345:517-25).

In the past, anti-inflammatory treatment was attempted by seeing the etiology of pulmonary fibrosis as inflammation, but the actual effect was insignificant (Annals of Internal Medicine 2001, 134:136-51; Chest 1996, 110:1058-67). TGF-β1 signaling leading to epithelial-mesenchymal transition (EMT) or EMT is being addressed as new pathogenesis and therapeutic goal (Annals of Internal Medicine 2001, 134:136-51; Chest 2007, 132:1311-21; The Journal of Clinical Investigation 2009, 119:213-24).

The main factor inducing EMT is TGF-β1. TGF-β1 induces the neogenesis of α-SMA (α-Smooth muscle actin) during EMT, and the neogenesis of α-SMA changes lung fibroblasts into myofibroblast phenotype. The expression of these myofibroblasts is estimated to play a major role in the development and progression of pulmonary fibrosis because the expression of these myofibroblasts is upregulated and continuously expressed at the site where lung fibrosis is progressing (Chest 2002,122:286S-9S).

In particular, it is known that the expression of α-SMA, vimentin, fibronectin, and SMA2/3 is increased in association with the TGF-β pathway, and E-cadherin is converted to N-cadherin, promoting EMT and causing pulmonary fibrosis (The Journal of Clinical Investigation 2009,119:213-24; Nat Rev Mol Cell Biol 2006,7:131-42; Cell Biol Int 2002,26:463-76). In addition, it is known that the Smad-dependent pathway and the Smad-independent mitogen-activated protein kinases (MAPK) pathway are involved in a cell by TGF-β1 (J Am Soc Nephrol 2002, 13:1464-72; Proc Natl Acad Sci USA 2001, 98:6686-91; Cancer Res 2001;61:4222-8). Furthermore, NADPH oxidases (NOX) such as NOX2 and NOX4 have been known to be involved in cell activation by TGF-β1 (Thorax. 2010 Aug;65(8):733-8). While oxidative stress is also known to be a factor in the progression of pulmonary fibrosis, bleomycin is known to increase oxidative stress by promoting nitric oxide (NO) synthesis in the lungs and to increase NOX expression through phosphorylation of MAPK signaling pathway factors (Scientific Reports 7(1):2252, 2017).

In addition, hypoxia-inducible factor-1α (HIF-1α) and epidermal growth factor receptor (EGFR) signaling are also known to be related to pulmonary fibrosis and have been proposed as targets for the treatment of pulmonary fibrosis (Am J Respir Cell Mol Biol. 2018 Feb; 58(2):216-231; Am J Physiol Lung Cell Mol Physiol. 2019 Jun 1;316(6):L1025-L1034). In particular, in vascular smooth muscle cells, EGFR promotes NO production and NOX expression through increased expression of the extracellular signal-regulated kinase (ERK) and AKT, one type of serine/threonine protein kinase, and phosphorylation of the downstream signal transducers thereof (Antioxidants & Redox Signaling 22(1):29-47, 2015). Additionally, EGFR phosphorylation is known to accelerate fibrosis by activating the MAPK signaling pathways, such as ERK1/2, P38, c-Jun N-terminal kinases (JNK), etc. (Journal of Respiratory Cell and Molecular Biology, 50(4): 723-736, 2014). Therapeutics, such as gefitinib, which are EGFR tyrosine kinase inhibitors, inhibit EGFR phosphorylation and thus inhibit fibroblast proliferation and extracellular deposition of collagen (Am J Respir Cell Mol Biol. 2006, 174(5):550-556; Am J Physiol Lung Cell Mol Physiol. 2008., 294(6):L1217-L1225).

Steroids, immunosuppressive agents, and anti-viral cytokines are typically used to treat pulmonary fibrosis, but according to the 2011 ATS/ERS Guidelines for Idiopathic Pulmonary Fibrosis, steroids and combination therapy with the immunosuppressant azathioprine has been shown to increase mortality.

In pulmonary fibrosis, alveolar epithelial cells and fibroblasts or myofibroblasts are evaluated as the main factors of the pathogenesis, and research is in progress on new drugs targeting these cells. Additionally, as the causes of pulmonary fibrosis are diverse, newly developed drugs are being developed to inhibit various pathways that cause fibrosis or inhibit the upstream signaling system rather than a single pathway.

The present disclosure discloses a novel flavone derivative having EMT inhibitory activity by inhibiting the TGF-β1 signaling pathway and use thereof for improving pulmonary fibrosis.

### Disclosure

### Technical Problem

An objective of the present disclosure is to provide a novel flavone derivative.

Another object of the present disclosure is to provide a composition for improving pulmonary fibrosis using a novel flavone derivative.

Other or specific objectives of the present disclosure will be presented below.

### Technical Solution

As confirmed in the following examples and experimental Examples, the present disclosure was completed by confirming that novel flavone derivatives of Formula 1 to 3 inhibit the TGF-β1 signaling pathway without showing specific cytotoxicity to A549 cells, derived from lung cancer, Hulec-5a cells, derived from pulmonary vessels, and human lung fibroblasts (HLF), thereby inhibiting epithelial-mesenchymal transition (EMT), and also show the effect of regulating the expression of pulmonary fibrosis-related factors (fibronectin, α-SMA, HIF-1α, NOX2, NOX4, and the like) in the A549 cells and Hulec-5a cells treated with epidermal growth factor (EGF) or bleomycin, which causes pulmonary fibrosis. 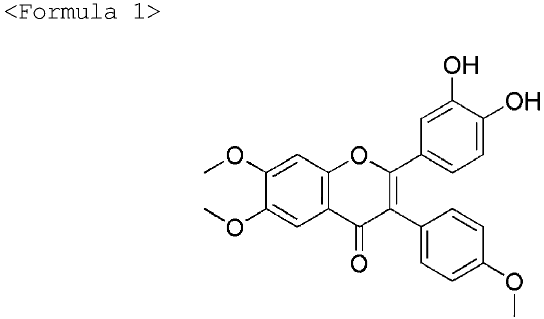
2-(3,4-dihydroxyphenyl)-6,7-dimethoxy-3-(4-methoxyphenyl)-4H-chromen-4-one 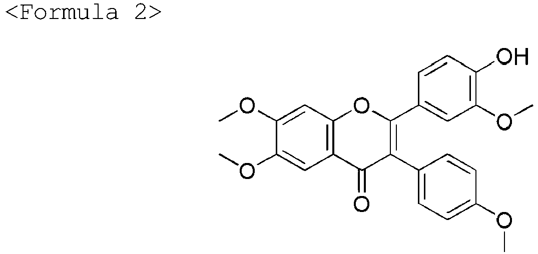
2-(4-hydroxy-5-methoxyphenyl)-6,7-dimethoxy-3-(4-methoxyphenyl)-4H-chromen-4-one 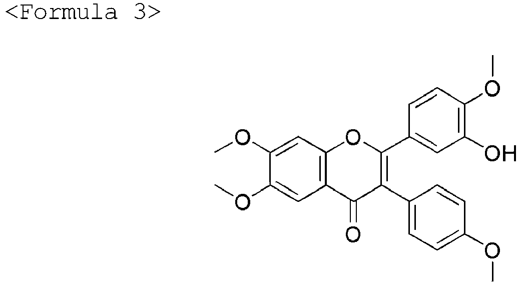
2-(4-methoxy-5-hydroxyphenyl)-6,7-dimethoxy-3-(4-methoxyphenyl)-4H-chromen-4-one

The present disclosure is based on the results of the experiments described below. In one aspect of the present disclosure, proposed is a flavone derivative of Formula 4 below, a hydrate thereof, or a solvate thereof. Alternatively, proposed is a composition for improving pulmonary fibrosis, the composition containing the flavone derivative of Formula 4 below, the hydrate thereof, or the solvate thereof as an active ingredient.

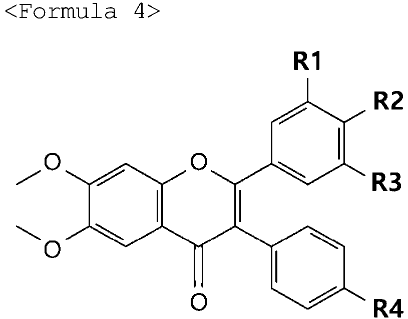

In Formula 4, R1, R2, R3, and R4 are each independently hydrogen (H), a hydroxy group (-OH), a methyl group (-CH₃), or a methoxy group (-OCH₃).

As used herein, the term "hydrate" refers to a compound to which water is bound and encompasses the meaning of inclusion compounds in which there is no chemical bond between water and a compound.

Additionally, as used herein, the term "solvate" refers to a compound formed between a molecule or ion of a solute and a molecule or ion of a solvent.

Additionally, as used herein, the term "active ingredient" refers to a component that may exhibit the desired activity by being used solely or may exhibit the desired activity by being used in conjunction with an inactive carrier.

In the composition of the present disclosure, the active ingredient may be included in a predetermined amount (effective amount) varying depending on the use thereof, formulation, etc., as long as the active ingredient may exhibit the effect of improving pulmonary fibrosis, etc., but the typical effective amount may be 0.001% to 99% by weight relative to the total weight of the composition. Here, the term "effective amount" refers to the amount included in the composition by which the intended medical and pharmacological effects, such as the mitigation of pulmonary fibrosis, can be obtained when the composition is administered to a mammal, preferably a human for a predetermined administration period suggested by a medical professional. Such an effective amount may be determined empirically by the ordinarily skilled in the art.

The composition of the present disclosure may be identified as a food composition in specific embodiments.

The food composition of the present disclosure may be prepared in any form. Specifically, the food composition may be prepared in the form of: beverages such as tea, juice, carbonated beverages, and ionic beverages; processed dairy products such as milk and yogurt; gums; snacks such as rice cakes, Korean sweets, bread, cake and confectionery, and noodles; and health functional food products such as tablets, capsules, pills, granules, liquids, powders, flakes, pastes, syrups, gels, jellies, bars, and the like. In addition, the food composition of the present disclosure may be categorized as any product class according to the legal and functional food classification confirming the enforcement regulations at the time of manufacture and distribution of the composition of the present disclosure. For example, it is a health functional food according to the "Health Functional Food Acts of Korea, or it may be confectionery, beans, multiples, beverages, or special-purpose foods according to each type of food according to the Food Code of Korea "Korean Food Sanitation Act" (Ministry of Food and Drug Safety Notice "Specifications and Standards of Food").

The food composition of the present disclosure may additionally contain food additives besides the active ingredient. Food additives are generally understood as substances added to and mixed with or infiltrated into food in manufacturing, processing, or preserving food. Since the food additives will be taken daily and for a long time along with food, the safety of the food additives must be guaranteed. Safe food additives are limitedly specified in terms of ingredients or functions in the Food Additives Ordinance in accordance with the laws for regulating the manufacture and distribution of food (for example, the "Food Sanitation Act" in Korea) in each country. In the Korean Food Additives Code (Ministry of Food and Drug Safety Notice "Food Additive Standards and Specifications"), food additives are classified into chemically synthetic products, natural additives, and mixed preparations in terms of ingredients. These food additives are classified into sweeteners, flavoring agents, preservatives, emulsifiers, acidulants, thickeners, and the like in terms of functions.

The sweetener is used to impart appropriate sweetness to food, and both natural and synthetic sweeteners may be used in the food composition of the present disclosure. Preferably, a natural sweetener is used. Examples of natural sweeteners include sugar sweeteners such as corn syrup solids, honey, sucrose, fructose, lactose, and maltose.

Flavoring agents are used to improve taste or fragrance, and both natural and synthetic flavoring ones may be used. Preferably, a natural flavoring agent is used. In the case of using natural flavoring agents, the purpose of nutritional enhancement, in addition to the purpose of adding flavor, may also be combined. The natural flavoring agent may be obtained from apples, lemons, tangerines, grapes, strawberries, peaches, or the like, or obtained from green tea leaves, horseradish leaves, bamboo leaves, cinnamon, chrysanthemum leaves, jasmine, and the like. In addition, those obtained from ginseng (red ginseng), bamboo shoots, aloe vera, ginkgo biloba, and the like may be used. The natural flavoring agent may be a liquid concentrate or a solid extract. In some cases, a synthetic flavoring agent may be used, and examples thereof may include esters, alcohols, aldehydes, terpenes, and the like.

Calcium sorbate, sodium sorbate, potassium sorbate, calcium benzoate, sodium benzoate, potassium benzoate, ethylenediaminetetraacetic acid (EDTA), and the like may be used as preservatives, and acacia gum, carboxymethylcellulose, xanthan gum, pectin, and the like may be used as emulsifiers. Citric acid, malic acid, fumaric acid, adipic acid, phosphoric acid, gluconic acid, tartaric acid, ascorbic acid, acetic acid, phosphoric acid, and the like may be used as the acidulant. The acidulant may be added so that the food composition has an appropriate acidity for inhibiting the growth of microorganisms, in addition to the purpose of enhancing the taste. As a thickening agent, a suspension implementation agent, a settling agent, a gel-forming agent, a bulking agent, and the like may be used.

The food composition of the present disclosure may contain, in addition to the food additives as described above, bioactive substances or minerals known in the art to supplement and reinforce functionality and nutrition and guaranteed stability as food additives.

Examples of such physiologically active substances may include catechins contained in green tea and the like, vitamins such as vitamin B1, vitamin C, vitamin E, and vitamin B12, tocopherol, dibenzoyl thiamine, and the like. As minerals, calcium preparations such as calcium citrate, magnesium preparations such as magnesium stearate, iron preparations such as iron citrate, chromium chloride, potassium iodide, selenium, germanium, vanadium, zinc, and the like may be included.

In the food composition of the present disclosure, the food additives described above may be included in an appropriate amount to achieve the purpose of the addition according to the type of product.

In relation to other food additives that may be included in the food composition of the present disclosure, reference may be made to the Food Ordinance or Food Additives Code according to each country's laws.

The composition of the present disclosure may be regarded as a pharmaceutical composition in another specific embodiment.

The pharmaceutical composition of the present disclosure may be prepared as an oral dosage form or parenteral dosage form according to the route of administration by a conventional method known in the art, including a pharmaceutically acceptable carrier, in addition to the active ingredient. Here, "pharmaceutically acceptable" means that it does not inhibit the activity of an active ingredient and does not have toxicity beyond what the application (prescription) target may adapt.

When the pharmaceutical composition of the present disclosure is prepared as an oral dosage form, it may be prepared in a formulation such as powders, granules, tablets, pills, sugar-coated tablets, capsules, liquids, gels, syrups, suspensions, wafers, according to methods known in the art together with suitable carriers. In this case, examples of suitable pharmaceutically acceptable carriers may include sugars such as lactose, glucose, sucrose, dextrose, sorbitol, mannitol, and xylitol, starches such as corn starch, potato starch, and wheat starch, celluloses such as cellulose, methylcellulose, ethylcellulose, sodium carboxymethylcellulose, and hydroxypropyl methylcellulose, polyvinylpyrrolidone, water, methyl hydroxybenzoate, propyl hydroxybenzoate, magnesium stearate, mineral oil, malt, gelatin, talc, polyols, vegetable oil, and the like. For formulation of the composition, diluents and/or excipients such as fillers, thickeners, binders, wetting agents, disintegrating agents, and surfactants may be used as necessary.

When the pharmaceutical composition of the present disclosure is prepared for parental use, it may be formulated in eye drops, injections, transdermal administrations, nasal inhalations (preparations for directly delivering drugs into the nasal cavity, oral cavity, respiratory tract, bronchial tubes, and the like using nebulizers), or suppositories together with a suitable carrier according to methods known in the art. When formulated as eye drops, as a suitable carrier, sterilized water, saline, 5% dextrose, and the like may be used, and benzalkonium chloride, methylparaben, ethylparaben, etc. may be added for preservative purposes as necessary. When formulated as an injection, sterilized water, polyols such as ethanol, glycerol, propylene glycol, or a mixture thereof may be used as a suitable carrier, and preferably, an IV solution, a phosphate-buffered saline (PBS) containing triethanol amine, an isotonic solution such as 5% dextrose, or the like may be used. When formulated for transdermal administrations, it may be formulated in the form of ointments, creams, lotions, gels, external solutions, pastes, liniments, aerosols, and the like. In the case of nasal inhalants, suitable propellants such as dichlorofluoromethane, trichlorofluoromethane, dichlorotetrafluoroethane, and carbon dioxide may be used to form an aerosol spray. When formulated as a suppository, Witepsol^{®}, tween 61, polyethylene glycols, cacao oil, laurin oil, polyoxyethylene sorbitan fatty acid esters, polyoxyethylene stearates, sorbitan fatty acid esters, and the like may be used as base agents.

Specific formulations of pharmaceutical compositions are known in the art, and references may refer to, for example, Remington's Pharmaceutical Sciences (19th ed., 1995). This document is considered a part of this disclosure.

A preferred dosage of the pharmaceutical composition of the present disclosure may range from 0.001 mg/kg to 10 g/kg per day, preferably 0.001 mg/kg to 1 g, depending on the patient's condition, weight, sex, age, the severity of the patient, and the route of administration. Administration may be performed once a day or divided into several times a day. Such dosages should not be construed as limiting the scope of the disclosure in any respect.

### Advantageous Effects

As described above, according to the present disclosure, it is possible to provide a composition for improving pulmonary fibrosis using a novel flavone derivative that inhibits the TGF-β1 signaling pathway without showing specific cytotoxicity to A549 cells, derived from lung cancer, Hulec-5a cells, derived from pulmonary vessels, and human lung fibroblasts (HLF), thereby inhibiting epithelial-mesenchymal transition (EMT), and also shows the effect of regulating the expression of pulmonary fibrosis-related factors in the A549 cells and Hulec-5a cells treated with epidermal growth factor (EGF) or bleomycin, which causes pulmonary fibrosis. The composition of the present disclosure can be commercialized into foods, such as health functional foods, or drugs, such as pharmaceuticals.

### Description of Drawings

FIGS. 1 and 2 show 1H-NMR and 13C-NMR results of novel flavone derivative I, respectively;
FIG. 3 shows LC-MS spectrum of novel flavone derivative I;
FIGS. 4 to 6 show cytotoxicity evaluation results of novel flavone derivative I;
FIGS. 7 and 8 show an effect of novel flavone derivative I upon TGF-β1 treatment in A549 cells;
FIG. 9 shows an effect of novel flavone derivative I upon TGF-β1 treatment in Hulec-5a cells;
FIG. 10 shows an effect of novel flavone derivative I upon TGF-β1 treatment in HLF cells;
FIGS. 11 and 12 show an effect of novel flavone derivative I upon EGF treatment in A549 cells;
FIG. 13 shows an effect of novel flavone derivative I in A549 cells by bleomycin treatment;
FIG. 14 shows an effect of novel flavone derivative I in Hulec-5a cells by bleomycin treatment;
FIGS. 15 and 16 show 1H-NMR and 13C-NMR results of novel flavone derivative II, respectively;
FIGS. 17 to 19 show cytotoxicity evaluation results of novel flavone derivative II;
FIGS. 20 and 21 show an effect of novel flavone derivative II upon TGF-β1 treatment in A549 cells;
FIG. 22 shows an effect of novel flavone derivative II upon TGF-β1 treatment in Hulec-5a cells;
FIG. 23 shows an effect of novel flavone derivative II upon TGF-β1 treatment in HLF cells;
FIG. 24 shows an effect of novel flavone derivative II upon EGF treatment in A549 cells;
FIGS. 25 and 26 show 1H-NMR and 13C-NMR results of novel flavone derivative III, respectively;
FIGS. 27 to 29 show cytotoxicity evaluation results of novel flavone derivative III;
FIG. 30 shows an effect of novel flavone derivative III upon TGF-β1 treatment in A549 cells;
FIG. 31 shows an effect of novel flavone derivative III upon TGF-β1 treatment in Hulec-5a; and
FIG. 32 shows an effect of novel flavone derivative III upon TGF-β1 treatment in HLF cells.

### Best Mode

Hereinafter, the present disclosure will be described with reference to examples and experimental examples. However, the scope of the present disclosure is not limited by the following examples and experimental examples.

### <Example> Preparation of novel flavone derivatives I to III and pulmonary fibrosis improvement activity test thereof

### <Example 1> Preparation of novel flavone derivative I and pulmonary fibrosis improvement activity test

### 1. Preparation of novel flavone derivative I

### Step 1

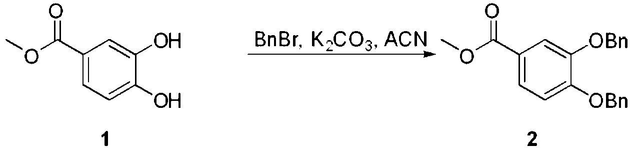

Potassium carbonate (K₂CO₃, 32.86 g, 238.10 mmol) and benzyl bromide (BnBr, 22.39 g, 130.95 mmol) were added to a solution of methyl 3,4-dihydroxybenzoate (1) (10 g, 59.52 mmol) in acetonitrile (100 mL) at 25°C and stirred for 5 hours at room temperature. After removing the solvent, the residue was diluted with 100 mL of water, extracted with ethyl acetate (100 mL x 2), dried over anhydrous sodium sulfate, and concentrated. The residue was purified by silica column chromatography (petroleum ether: ethyl acetate = 10:1) to obtain a yellow solid, methyl 3,4-bis(benzyloxy)benzoate (2) (19 g, yield: 91.73%).
LC-MS (ESI) *m*/*z* 349.2[M+1]⁺

### Step 2

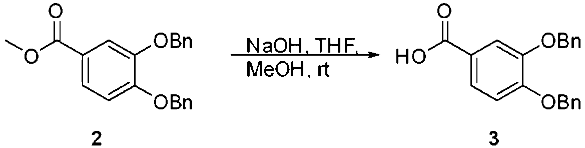

After adding a solution of 1 M NaOH (aq) (60 mL, 60.0 mmol) to the solution of methyl 3,4-bis(benzyloxy)benzoate (2) (19 g, 54.60 mmol) in THF/MeOH (50 mL/50 mL), the mixture was stirred at 20°C for 12 hours. The mixture was concentrated, diluted with water, acidified to a pH of 5 with 1 M HCl (aq), and then exacted with dichloromethane (DCM). The organic solvent was dried over Na₂SO₄, filtered, and concentrated to obtain a white solid, 3,4-bis(benzyloxy)benzoic acid (3) (13 g, crude).
LC-MS (ESI) *m*/*z* 335.2[M+1]⁺

### Step 3

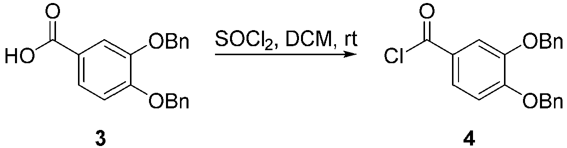

Thionyl chloride (4.99 g, 41.92 mmol) and DMF (15.33 mg, 0.21 mmol) were added to the solution of 3,4-bis(benzyloxy)benzoic acid **(3)** (7 g, 20.96 mmol) in DCM (50 mL) at 0°C and stirred at 0°C for 4 hours. The mixture was concentrated to obtain a yellow solid, 3,4-bis(benzyloxy)benzoyl chloride **(4)** (8 g, crude, which was used immediately for the next fourth step.

### Step 4

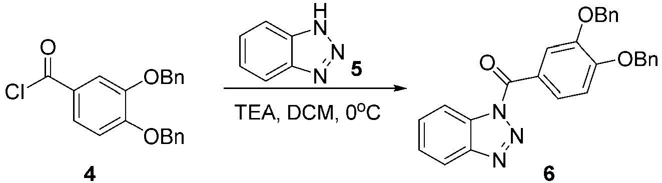

Triethylamine (TEA, 2.12 g, 20.96 mmol) and *1H-*benzo[d][1,2,3]triazole **(5)** (2.49 g, 20.96 mmol) were added to the solution of 3,4-bis(benzyloxy)benzoyl chloride **(4)** (8 g, 20.96 mmol) in DCM (50 mL) at 0°C and stirred at 0°C for 4 hours. After adding an aqueous ammonium chloride solution to the obtained product, the resulting product was extracted with ethyl acetate (EtOAc) three times. Then, the organic layers were washed with 3 M aqueous sodium hydroxide solution, dried over Na₂SO₄, concentrated under reduced pressure, and purified by recrystallization using n-hexane-DCM to obtain a white solid, (1H-benzo[d][1,2,3]triazol-1-yl) (3,4-bis(benzyloxy)phenyl)methanone **(6)** (8 g, yield: 87.74%).
LC-MS (ESI) *m*/*z* 436.2[M+1]⁺

### Step 5

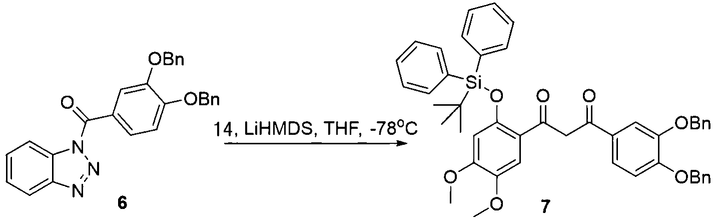

Lithium bis(trimethylsilyl)amide (LiHMDS, CAS No. 4939-32-1) (55 mL, 55 mmol, 1 M solution in THF) was added to a solution of 1-(2-((tert-butyldiphenylsilyl)oxy)-4,5-dimethoxyphenyl)ethan-1-one (8 g, 18.43 mmol) **(14,** prepared in the following 11th step) in THF (50 mL) at -70°C and stirred for 1 hour at -70°C. Then, the solution of (1H-benzo[d][1,2,3]triazol-1-yl)(3,4-bis(benzyloxy)phenyl)methanone **(6)** (8 g, 18.43 mmol) in 30 mL of THF at -70°C was added thereto. Next, the resulting mixture was stirred for 2 hours at 0°C, added to a saturated aqueous ammonium chloride solution, and extracted with ethyl acetate (EtOAc) three times. Subsequently, the organic layers were dried over Na₂SO₄ and concentrated under reduced pressure to obtain a yellow oil, 1-(3,4-bis(benzyloxy)phenyl)-3-(2-((tert-butyldiphenylsilyl)oxy)-4,5-dimethoxyphenyl)propane-1,3-dione **(7)** (19 g, crude), which was used immediately for the next sixth step.

### Step 6

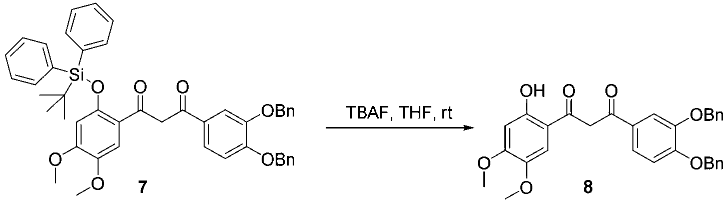

Tetrabutylammonium fluoride (TBAF, 5.23 g, 20.00 mmol) was added to the solution of 1-(3,4-bis(benzyloxy)phenyl)-3-(2-((tert-butyldiphenylsilyl)oxy)-4,5-dimethoxyphenyl)propane-1,3-dione **(7)** (19 g, crude) in THF (50 mL) and stirred at 20°C for 3 hours. The resulting mixture was added to a saturated aqueous ammonium chloride solution and extracted with EtOAc three times. Then, the organic layers were dried over Na₂SO₄ and concentrated under reduced pressure. The resulting product was purified by silica column chromatography (petroleum ether:ethyl acetate = 4:1) to obtain a yellow solid, 1-(3,4-bis(benzyloxy)phenyl)-3-(2-hydroxy-4,5-dimethoxyphenyl)propane-1,3-dione **(8)** (5.3 g, yield: 56.17%).
LC-MS (ESI) m/z 513.3[M+1] ⁺

### Step 7

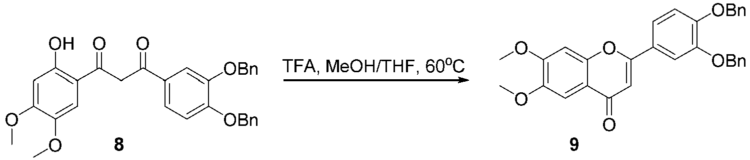

TFA (7.92 mL, 103.50 mmol) was added to the solution of 1-(3,4-bis(benzyloxy)phenyl)-3-(2-hydroxy-4,5-dimethoxyphenyl)propane-1,3-dione **(8)** (5.3 g, 10.35 mmol) in MeOH/THF = 1/5 (30 mL) at 0°C and stirred at 60°C for 5 hours. The solvent was removed and purified by recrystallization using n-hexane-DCM to obtain a white solid, 2-(3,4-bis(benzyloxy)phenyl)-6,7-dimethoxy-4H-chromen-4-one **(9)** (3 g, yield: 58. 68%) .
LC-MS (ESI) *m*/*z* 495.2[M+1]⁺

### Step 8

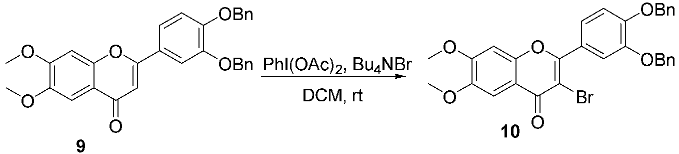

Tetrabutylammonium bromide (TBAB, 2.93 g, 9.11 mmol) was added to a solution of PhI(OAc)₂ (2.93 g, 9.11 mmol) in DCM (30 mL) and stirred at 20°C for 1 hour under a nitrogen atmosphere. Next, the resulting mixture was added to the solution of 2-(3,4-bis(benzyloxy)phenyl)-6,7-dimethoxy-4H-chromen-4-one **(9)** (3 g, 6.07 mmol) in anhydrous DCM (20 ml) and stirred at 20°C for 8 hours. Then, the resulting mixture was added to a saturated aqueous ammonium chloride solution and extracted with DCM three times. Subsequently, the organic solvent was removed using Na₂SO₄, concentrated under reduced pressure, and purified by silica column chromatography (petroleum ether: ethyl acetate = 2:1) to obtain a yellow solid, 2-(3,4-bis(benzyloxy)phenyl)-3-bromo-6,7-dimethoxy-4H-chromen-4-one **(10)** (3.2 g, yield: 92.16%).
LC-MS (ESI) m/z 575.2[M+1]⁺

### Step 9

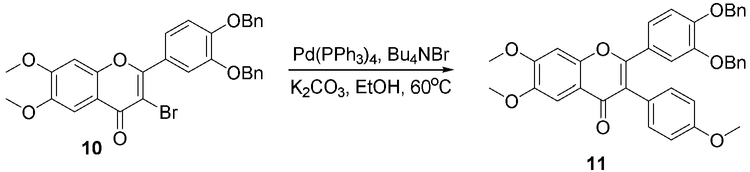

TBAB (1.80 g, 5.59 mmol), K₂CO₃ (1.54 g, 11.18 mmol), and Pd(PPh₃)₄ (347 mg, 0.30 mmol) were added to the solution of 2-(3,4-bis(benzyloxy)phenyl)-3-bromo-6,7-dimethoxy-4H-chromen-4-one **(10)** (3.2 g, 5.59 mmol) in THF/EtOH = 1/1 (30 mL) and stirred at 80°C for 5 hours under a nitrogen atmosphere. Next, the solvent was removed and purified by silica column chromatography (petroleum ether:ethyl acetate = 1:1) to obtain a yellow solid, 2-(3,4-bis(benzyloxy)phenyl)-6,7-dimethoxy-3-(4-methoxyphenyl)-4H-chromen-4-one **(11)** (2.3 g, yield: 68.57%) .
LC-MS (ESI) *m*/*z* 601.3[M+1] ⁺

### Step 10

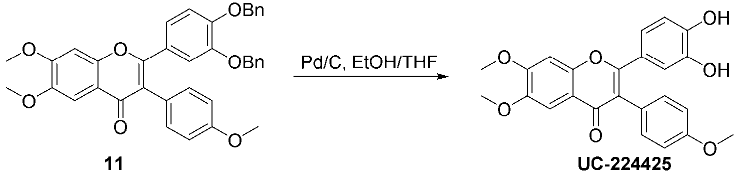

Pd/C (500 mg) was added to the solution of 2-(3,4-bis(benzyloxy)phenyl)-6,7-dimethoxy-3-(4-methoxyphenyl)-4H-chromen-4-one **(11)** (1.6 g, 2.67 mmol) in THF/EtOH = 1/1 (30 mL) and stirred overnight at room temperature under an H₂ condition. The reaction mixture was filtered through Celite, and the filtrate was concentrated. Then, the concentrate was purified by recrystallization using diethyl ether (Et₂O) to obtain a green solid as novel flavone derivative I **(Achem I),** 2-(3,4-dihydroxyphenyl)-6,7-dimethoxy-3-(4-methoxyphenyl)-4H-chromen-4-one **(UC-224425)** (750 mg, yield: 66.88%), which was the end product.

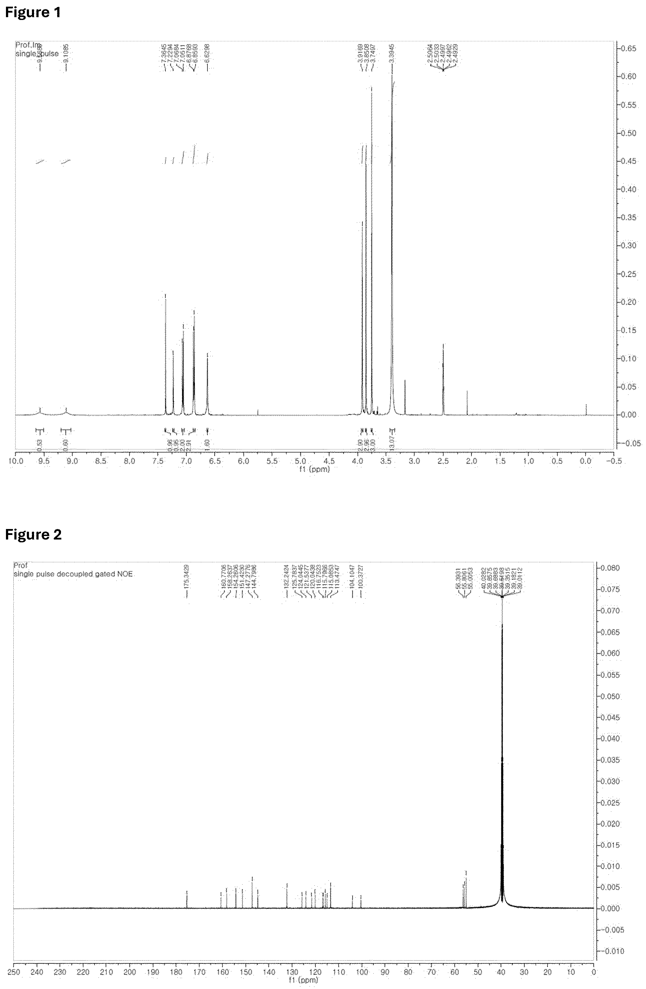

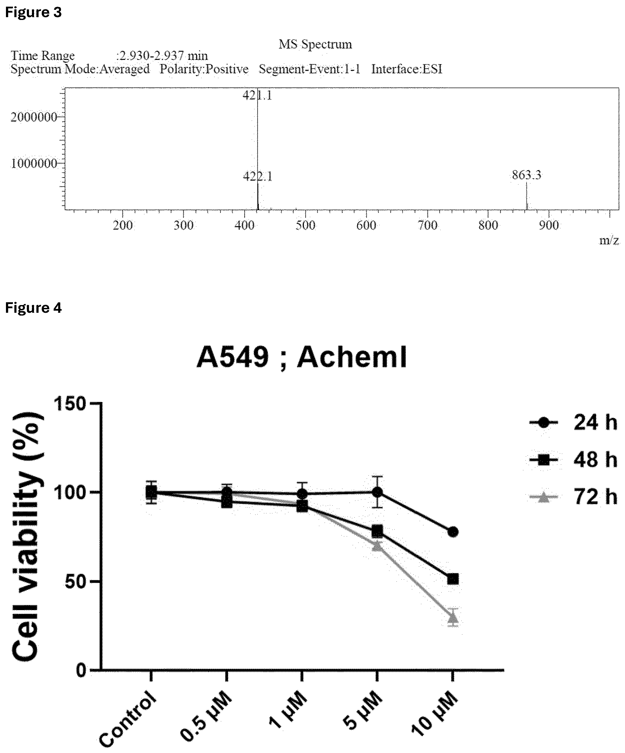

FIGS. 1 and 2 show the ¹H NMR (DMSO-d6) and ¹³C NMR (DMSO-d6) results, respectively, and FIG. 3 shows LC-MS data (LC-MS (ESI) *m*/*z* 421.1[M+1]⁺).

### Step 11

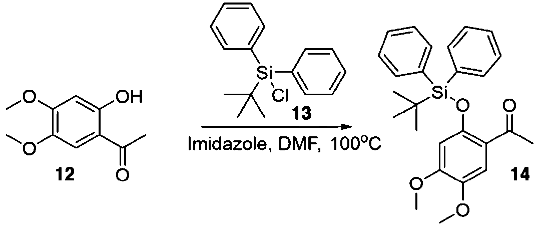

Imidazole (624 mg, 9.18 mmol) and tert-butylchlorodiphenylsilane **(13)** (3.0 g, 11.2 mmol) were added to 1-(2-hydroxy-4,5-dimethoxyphenyl)ethan-1-one **(12)** (1 g, 5.1 mmol) in DMF (5 mL) at 0°C and stirred at 100°C for 10 hours. The resulting mixture was added to a saturated ammonium chloride aqueous solution and extracted with DCM three times. Next, the organic layers were dried over Na₂SO₄, concentrated under reduced pressure, and purified by recrystallization using n-hexane-EA to obtain a white solid, 1-(2-((tert-butyldiphenylsilyl)oxy)-4,5-dimethoxyphenyl)ethan-1-one **(14)** (800 mg, yield: 87.74%). The compound obtained in such a manner was used as the reactant in the fifth step described above.

### 2. Pulmonary fibrosis improvement activity test of novel flavone derivative I

### 2.1 Evaluation of cytotoxicity

### 2.1.1 Evaluation of cytotoxicity to 549 cells

To confirm the effect of novel flavone derivative I on cell viability, A549 cells, derived from lung cancer, were used to examine cell viability with MTT [3-(4,5-dimethylthiazol-2-yl)-2,5-diphenyltetrazolium bromide]. After being suspended in a medium, A549 cells were dispensed into a 96-well plate and incubated for 24 hours. After 24 hours, 200 µL of a medium containing 0 to 20 µM BGC was added to each well of the plate and incubated for 24 or 48 hours. After incubation, MTT was diluted (0.5 mg/ml) in phosphate-buffered saline (PBS). Then, 20 µL of the diluted MTT was dispensed into each well, and incubation was performed in a CO2 incubator for 4 hours. After 4 hours, the MTT solution was removed, and 200 µL of dimethyl sulfoxide (DMSO) was dispensed into each well to dissolve the formazan precipitate for 5 minutes. Next, the absorbance was measured at 595 nm.

FIG. 4 shows cell viability after 24, 48, and 72 hours as a percentage compared to the control group, the untreated sample group. No cytotoxicity was observed at a treatment concentration of 5 µM when incubated for 24 hours, and about 80% of cell viability was shown at a treatment concentration of 5 µM when incubated for 48 hours.

### 2.1.2 Evaluation of cytotoxicity to HULEC-5a cells

To confirm the effect of novel flavone derivative I on cell viability, HULEC-5a, human lung microvascular endothelial cells, were used to examine cell viability with MTT [3-(4,5-dimethylthiazol-2-yl)-2,5-diphenyltetrazolium bromide]. After being suspended in a medium, HULEC-5a cells were dispensed into a 96-well plate and incubated for 24 hours. After 24 hours, 200 µL of a medium containing 0 to 10 µM novel flavone derivative I was added to each well of the plate and incubated for 24, 48, and 72 hours. After incubation, all media was removed, and MTT was diluted (0.5 mg/mL) in phosphate-buffered saline (PBS). Then, 200 µL of the diluted MTT was dispensed into each well, and incubation was performed in a CO₂ incubator for 4 hours. After 4 hours, the MTT solution was removed, and 160 µL of dimethyl sulfoxide (DMSO) was dispensed into each well to dissolve the formazan precipitate for 30 minutes. Next, the absorbance was measured at 570 nm.

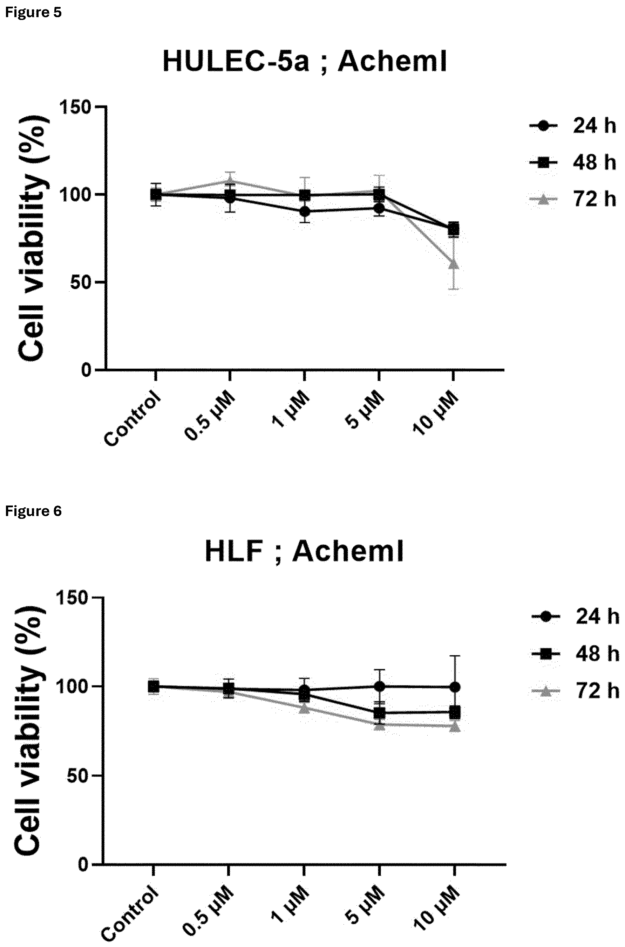

FIG. 5 shows cell viability after 24, 48, and 72 hours as a percentage compared to the control group, the untreated sample group. No cytotoxicity was observed at treatment concentrations of up to 5 µM when incubated for 24, 48, and 72 hours.

### 2.1.3 Evaluation of cytotoxicity to HLF cells

To confirm the effect of novel flavone derivative I on cell viability, human lung fibroblasts (HLF) were used to examine cell viability with MTT [3-(4,5-dimethylthiazol-2-yl)-2,5-diphenyltetrazolium bromide]. After being suspended in a medium, HLF cells were dispensed into a 96-well plate and incubated for 24 hours. After 24 hours, 200 µL of a medium containing 0 to 10 µM novel flavone derivative I was added to each well of the plate and incubated for 24, 48, and 72 hours. After incubation, all media was removed, and MTT was diluted (0.5 mg/mL) in phosphate-buffered saline (PBS). Then, 200 µL of the diluted MTT was dispensed into each well, and incubation was performed in a CO₂ incubator for 4 hours. After 4 hours, the MTT solution was removed, and 160 µL of dimethyl sulfoxide (DMSO) was dispensed into each well to dissolve the formazan precipitate for 30 minutes. Next, the absorbance was measured at 570 nm.

FIG. 5 shows cell viability after 24, 48, and 72 hours as a percentage compared to that of the control group, the untreated sample group. About 80% or higher level of cell viability was shown at all treatment concentrations when incubated for 24, 48, and 72 hours.

The incubation time and sample treatment concentration in subsequent tests took into account the cytotoxicity test results.

### 2.2 Effect upon TGF-β1 treatment in A549 cells

### (1) Test method

3.5 * 10⁵ A549 cells, derived from lung cancer, were suspended in a medium, dispensed into a 60-mm plate, and incubated for 48 hours. After 48 hours of incubation, each plate was treated with TGF-β1 (10 ng/ml) alone or in combination with novel flavone derivative I at varying concentrations or a predetermined concentration and/or nintedanib (NIDB), a therapeutic drug for idiopathic pulmonary fibrosis and interstitial lung disease as a positive control. After 48 hours of incubation, the cells were lysed with RIPA lysis buffer ([50 mM Tris]-Cl (pH, 7.4), 1% NP-40, 150 mM NaCl, 1 mM EDTA, and 1 mM phenylmethylsulfonyl fluoride (PMSF)) containing MG-132 (10 µM, proteasome inhibitor, protect the HIF-1α subunit from proteasome degradation). Next, only the supernatant was collected by centrifugation (at 14,000 rpm for 10 minutes) at 4°C, and the proteins were separated by SDS-polyacrylamide gel electrophoresis (SDS-PAGE). The separated proteins were transferred to a polyvinylidene difluoride membrane (PVDF membrane), reacted in 5% nonfat dry milk for 3 hours, and then reacted with primary antibodies at 4°C for 12 hours. After 12 hours, the proteins further reacted with secondary antibodies corresponding to the respective primary antibodies. Then, the protein band was examined, according to the manufacturer's protocol, using an ECL kit.

### (2) Test results

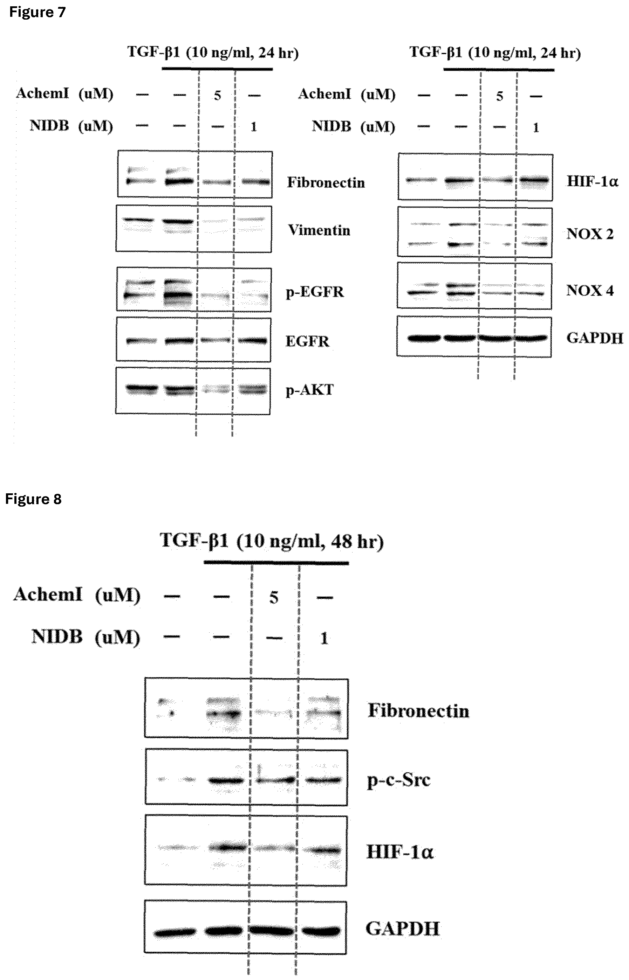

FIGS. 7 and 8 show the test results.

Referring to FIG. 7, when treating the A549 cells with 10 ng/mL of TGF-β1 and 5 µM of novel flavone derivative I for 24 hours, the results thereof showed a tendency that the levels of fibronectin, HIF-1α, NOX2, NOX4, vimentin, p-EGFR, EGFR, and p-AKT proteins, increased by TGF-β1 treatment, were decreased by novel flavone derivative I.

Additionally, referring to FIG. 8, when treating the A549 cells with 10 ng/mL of TGF-β1 and 5 µM of novel flavone derivative I for 48 hours, the results thereof showed a tendency that the levels of fibronectin, HIF-1α, and p-c-Src proteins, increased by TGF-β1 treatment, were decreased by novel flavone derivative I.

TGF-β1 is a factor used as a typical fibrosis-promoting cytokine in both type II alveolar epithelial cells in rats and humans. The fact that in the presence of TGF-β1, epithelial cells are first transformed into mesenchymal cells, then into fibroblasts, and eventually into myofibroblasts is known through several studies in the art (TGF-β-Induced Endothelial-Mesenchymal Transition in Fibrotic Diseases, Int J Mol Sci., 2017,18(10):2157-2179).

### 2.3 Effect upon TGF-β1 treatment in Hulec-5a cells

### (1) Test method

1.2 × 10⁶ Hulec-5a cells, derived from pulmonary vessels, were suspended in a medium, dispensed into a 100-mm plate, and incubated for 24 hours. After 24 hours of incubation, each plate was treated with TGF-β1 (10 ng/ml) alone or in combination with novel flavone derivative I at varying concentrations or a predetermined concentration and/or nintedanib (NIDB), a therapeutic drug for idiopathic pulmonary fibrosis and interstitial lung disease. After 3, 24, and 48 hours of incubation, the cells were lysed with RIPA lysis buffer ([50 mM Tris]-Cl (pH, 7.4), 1% NP-40, 150 mM NaCl, 1 mM EDTA, and 1 mM phenylmethylsulfonyl fluoride (PMSF)) containing MG-132 (10 µM, proteasome inhibitor, protect the HIF-1α subunit from proteasome degradation). Next, only the supernatant was collected by centrifugation (at 13,000 rpm for 7 minutes) at 4°C, and the proteins were separated by SDS-polyacrylamide gel electrophoresis (SDS-PAGE). The separated proteins were transferred to a polyvinylidene difluoride membrane (PVDF membrane), reacted in 5% nonfat dry milk for 2 hours, and then reacted with primary antibodies at 4°C overnight. After overnight, the proteins further reacted with secondary antibodies corresponding to the respective primary antibodies. Then, the protein band was examined, according to the manufacturer's protocol, using an ECL kit.

### (2) Test results

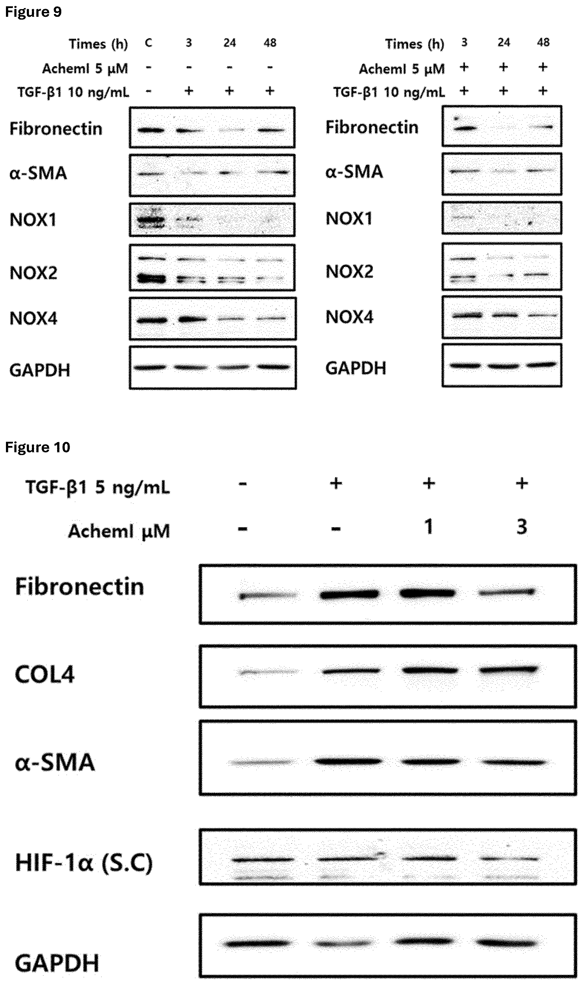

FIG. 9 shows the result thereof. Referring to FIG. 9, when treating the HULEC-5a cells with 10 ng/mL of TGF-β1 and/or 5 µM of novel flavone derivative I for 3, 24, and 48 hours, the results thereof showed a tendency that the levels of fibrosis-related factors (Fibronectin, α-SMA, NOX1, NOX2, and NOX4), increased by TGF-β1 treatment, were decreased by the treatment with novel flavone derivative I.

### 2.4 Effect upon TGF-β1 treatment in HLF cells

### (1) Test method

After suspending 5 × 10⁶ human lung fibroblast (HLF) cells in a medium, 1.2 × 10⁶ Hulec-5a cells, derived from pulmonary vessels, were suspended in the medium, dispensed into a 100-mm plate, and incubated for 24 hours. After 24 hours of incubation, each plate was treated with TGF-β1 (10 ng/ml) alone or in combination with novel flavone derivative I at varying concentrations or a predetermined concentration and/or nintedanib (NIDB), a therapeutic drug for idiopathic pulmonary fibrosis and interstitial lung disease. After 24 and 48 hours of incubation, the cells were lysed with RIPA lysis buffer ([50 mM Tris]-Cl (pH, 7.4), 1% NP-40, 150 mM NaCl, 1 mM EDTA, and 1 mM phenylmethylsulfonyl fluoride (PMSF)) containing MG-132 (10 µM, proteasome inhibitor, protect the HIF-1α subunit from proteasome degradation). Next, only the supernatant was collected by centrifugation (at 13,000 rpm for 7 minutes) at 4°C, and the proteins were separated by SDS-polyacrylamide gel electrophoresis (SDS-PAGE). The separated proteins were transferred to a polyvinylidene difluoride membrane (PVDF membrane), reacted in 5% nonfat dry milk for 2 hours, and then reacted with primary antibodies at 4°C overnight. After overnight, the proteins further reacted with secondary antibodies corresponding to the respective primary antibodies. Then, the protein band was examined, according to the manufacturer's protocol, using an ECL kit.

### (2) Test results

FIG. 10 shows the results thereof. Referring to FIG. 10, when treating the cells with 5 ng/mL of TGF-β1 and 1 and 3 µM of novel flavone derivative I for 24 hours, the results thereof showed a tendency that the levels of fibrosis-related factors (fibronectin, COL4, α-SMA, and HIF-1α), increased by TGF-β1 treatment, were decreased by novel flavone derivative I.

### 2.5 Effect upon EGF treatment in A549 cells

### (1) Test method

A549 cells, derived from lung cancer, were incubated at different cell numbers depending on the sample treatment time. 4.5 × 10⁵ cells (30 minutes to 12 hours), 4.0 × 10⁵ cells (24 hours), and 3.5 × 10⁵ cells (48 hours) were suspended in the respective media, dispensed into a 60-mm plate, and incubated for 24 hours. After 24 hours, each plate was treated with EGF (2 ng/ml) alone or in combination with novel flavone derivative I at varying concentrations or a predetermined concentration. After a predetermined period (30 minutes to 48 hours) of incubation, the cells were lysed with RIPA lysis buffer ([50 mM Tris]-Cl (pH, 7.4), 1% NP-40, 150 mM NaCl, 1 mM EDTA, and 1 mM, phenylmethylsulfonyl fluoride (PMSF)) containing MG-132 (10 µM). Next, only the supernatant was collected by centrifugation (at 14,000 rpm for 10 minutes) at 4°C, and the proteins were separated by SDS-polyacrylamide gel electrophoresis (SDS-PAGE). The separated proteins were transferred to a polyvinylidene difluoride membrane (PVDF membrane), reacted in 5% nonfat dry milk for 3 hours, and then reacted with primary antibodies at 4°C for 12 hours. After 12 hours, the proteins further reacted with secondary antibodies corresponding to the respective primary antibodies. Then, the protein band was examined, according to the manufacturer's protocol, using an ECL kit.

### (2) Test results

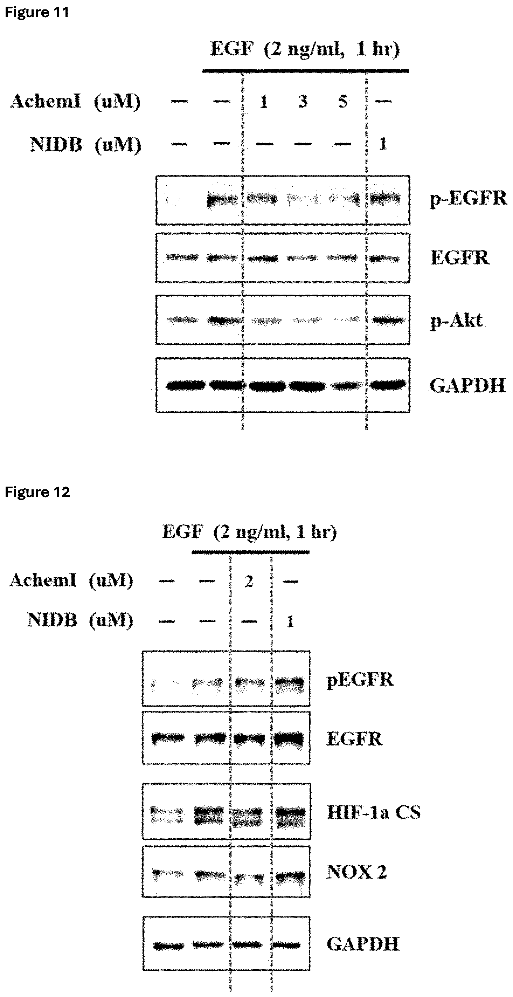

FIGS. 11 and 12 show the results thereof. EGF, a ligand of EGFR, has been reported to cause pulmonary fibrosis (Rapamycin prevents transforming growth factor-a-induced pulmonary fibrosis. Am J Respir Cell Mol Biol. 2009,41(5):562-572). Thus, the degree to which pulmonary fibrosis-related factors, whose expression levels were increased in A549 cells by EGF treatment, were inhibited by the treatment with novel flavone derivative I was evaluated.

When treating the A549 cells with 2 ng/mL of EGF and novel flavone derivative I at 1, 3, and 5 or 2 µM for 1 hour, the results thereof showed a tendency that the levels of fibrosis-related factors (p-EGFR, EGFR, p-AKT, HIF-1α, and NOX2), increased by EGF treatment, were decreased by novel flavone derivative I.

### 2.6 Effect of bleomycin treatment on A549 cells

### (1) Test method

A549 cells, derived from lung cancer, were incubated at different cell numbers depending on the sample treatment time. 4.5 × 10⁵ cells (30 minutes to 12 hours), 4.0 × 10⁵ cells (24 hours), and 3.5 × 10⁵ cells (48 hours) were suspended in the respective media, dispensed into a 60-mm plate, and incubated for 24 hours. After 24 hours, each plate was treated with bleomycin (BLM) alone or in combination with novel flavone derivative I at a predetermined concentration. After a predetermined period of incubation, the cells were lysed with RIPA lysis buffer ([50 mM Tris]-Cl (pH, 7.4), 1% NP-40, 150 mM NaCl, 1 mM EDTA, and 1 mM phenylmethylsulfonyl fluoride (PMSF)) containing MG-132 (10 µM). Next, only the supernatant was collected by centrifugation (at 14,000 rpm for 10 minutes) at 4°C, and the proteins were separated by SDS-polyacrylamide gel electrophoresis (SDS-PAGE). The separated proteins were transferred to a polyvinylidene difluoride membrane (PVDF membrane), reacted in 5% nonfat dry milk for 3 hours, and then reacted with primary antibodies at 4°C for 12 hours. After 12 hours, the proteins further reacted with secondary antibodies corresponding to the respective primary antibodies. Then, the protein band was examined, according to the manufacturer's protocol, using an ECL kit.

### (2) Test results

BLM is a substance used to establish animal models of pulmonary fibrosis and is known to induce abnormal extracellular matrix deposition and abnormal accumulation of fibroblasts in lesions. Additionally, BLM is a substance used to identify the molecular mechanism of pulmonary fibrosis or to screen therapeutics for pulmonary fibrosis (Cellular senescence and EMT crosstalk in bleomycin-induced pathogenesis of pulmonary fibrosis-an in vitro analysis, Cell Biol Int. 2020,44(2):477-487).

As shown in FIG. 3, when treating the A549 cells with BLM and/or novel flavone derivative I for 3, 24, and 48 hours, the results thereof showed a tendency that the levels of pulmonary fibrosis-related factors (fibronectin, α-SMA, HIF-1α, NOX1, NOX2, and NOX4), increased by BLM treatment, were decreased by treatment with novel flavone derivative I.

### 2.7 Effect of bleomycin treatment on Hulec-5a

### (1) Test method

1.2 × 10⁶ Hulec-5a cells, derived from pulmonary vessels, were suspended in a medium, dispensed into a 100-mm plate, and incubated for 24 hours. After 24 hours, each plate was treated with BLM alone or in combination with novel flavone derivative I at varying concentrations or a predetermined concentration. After a predetermined period of incubation, the cells were lysed with RIPA lysis buffer ([50 mM Tris]-Cl (pH, 7.4), 1% NP-40, 150 mM NaCl, 1 mM EDTA, and 1 mM phenylmethylsulfonyl fluoride (PMSF)) containing MG-132 (10 µM). Next, only the supernatant was collected by centrifugation (at 14,000 rpm for 10 minutes) at 4°C, and the proteins were separated by SDS-polyacrylamide gel electrophoresis (SDS-PAGE). The separated proteins were transferred to a polyvinylidene difluoride membrane (PVDF membrane), reacted in 5% nonfat dry milk for 3 hours, and then reacted with primary antibodies at 4°C for 12 hours. After 12 hours, the proteins further reacted with secondary antibodies corresponding to the respective primary antibodies. Then, the protein band was examined, according to the manufacturer's protocol, using an ECL kit.

### (2) Test results

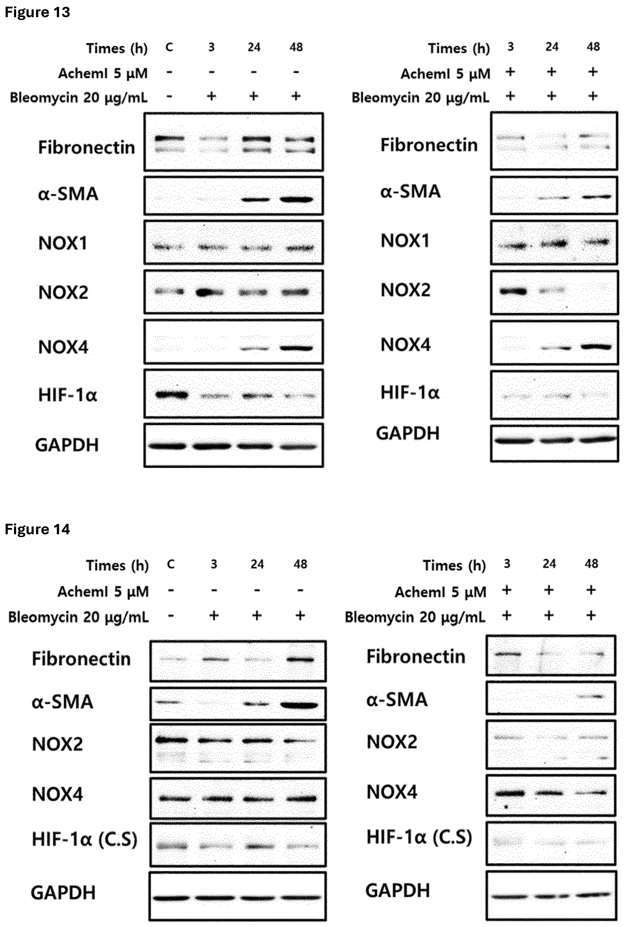

FIG. 14 shows the results thereof. Like the results of treating the A549 cells with bleomycin, when treating the Hulec-5a cells, derived from pulmonary vessels, with bleomycin, the results thereof showed a tendency that the levels of pulmonary fibrosis-related factors (fibronectin, α-SMA, HIF-1α, NOX2, and NOX4) were decreased by the treatment with novel flavone derivative I.

### <Example 2> Preparation of novel flavone derivative II and pulmonary fibrosis improvement activity test

### 1. Preparation of novel flavone derivative II

### Step 1

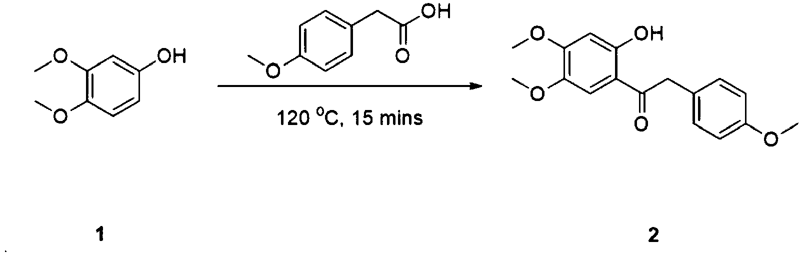

First, 2-(4-methoxyphenyl)acetic acid (25.6 g, 154 mmol) (CAS No: 104-01-8) was added to a solution of 3,4-dimethoxyphenol (1) (25 g, 162 mmol) in BF₃.Et₂O (40 mL) at room temperature under a nitrogen condition, heated to 120°C, and stirred for 15 minutes. The mixture was cooled to room temperature, poured into 100 mL of water, and extracted with EA (100 mL). The organic phases were combined, washed with brine, and dried over sodium sulfate. After being filtrated, the solution was concentrated under vacuum to obtain 50 g of crude oil, which was purified through silica column (PE/EA = 10% to 50%) to obtain 26 g of a white solid, 1-(2-hydroxy-4,5-dimethoxyphenyl)-2-(4-methoxyphenyl)ethenone (2) as (yield: 55%).

¹H NMR (400 MHz, CDCl₃) δ: 12.62 (s, 1H), 7.02 - 7.16 (m, 3H), 6.90 (d, J = 8.4 Hz, 2H), 6.45 (s, 1H), 4.15 (s, 1H), 3.90 (s, 3H), 3.82 (s, 3H), 3.79 (s, 3H).

### Step 2

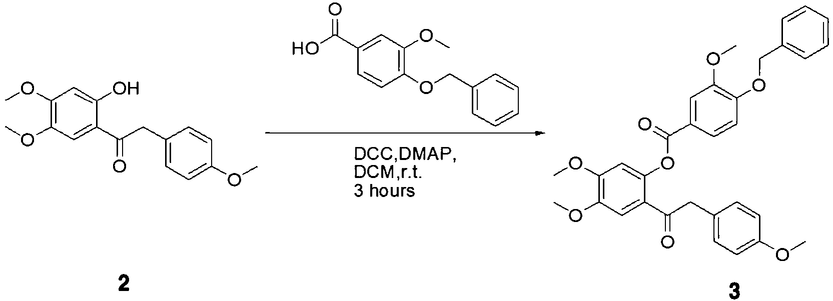

N,N'-dicyclohexylcarbodiimide (DCC) (21.2 g, 102.6 mmol) and dimethylaminopyridine (DMAP) (1.3 g, 10.6 mmol) were added to the solution of 1-(2-hydroxy-4,5-dimethoxyphenyl)-2-(4-methoxyphenyl)ethenone **(2)** (15.5 g, 51.3 mmol) and 4-(benzyloxy)-3-methoxybenzoic acid (15.9 g, 61.5 mmol) (CAS No.: 1486-53-9) in dichloromethane (DCM) (1000 mL). Then, the resulting mixture was stirred at room temperature for 3 hours and concentrated under vacuum pressure to obtain a white solid. The resulting solid was purified through silica column to obtain a target compound, which was 11 g of a white solid, 4,5-dimethoxy-2-(2-(4-methoxyphenyl)acetyl)phenyl 4-(benzyloxy)-3-methoxybenzoate **(3)** (yield: 38%).

¹H NMR (400 MHz, DMSO-*d*₆) δ: 7.50 - 7.24 (m, 7H), 7.15 - 7.05 (m, 4H), 6.97 - 6.92 (m, 2H), 6.90 (s, 1H), 5.12 (s, 2H), 3.96 (s, 3H), 3.90 (s, 3H), 3.78 (s, 3H), 3.51 (s, 3H).

### Step 3

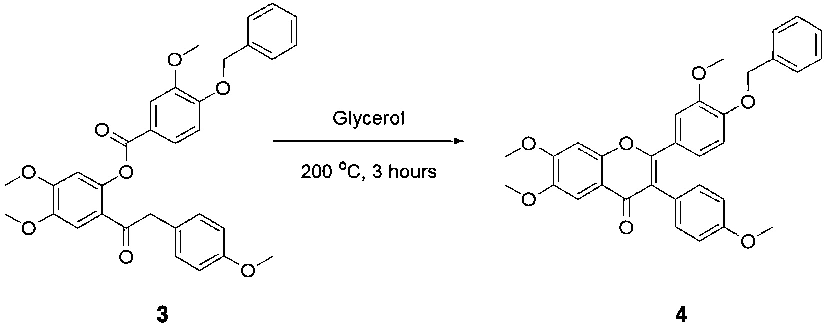

A mixture of 4,5-dimethoxy-2-(2-(4-methoxyphenyl) acetyl) phenyl 4-(benzyloxy)-3-methoxybenzoate **(3)** (3.2 g, 5.9 mmol) and glycerol (30 mL) was degassed with nitrogen three times and heated to 200°C for 3 hours. The resulting solution was cooled to room temperature, poured into 100 mL of water, and extracted with EA (100 mL). The organic phases were combined, washed with brine, dried over sodium sulfate, and concentrated to obtain a crude material, which was purified by silica column to obtain 800 mg of a white solid, 2-(4-(benzyloxy)-3-methoxyphenyl)-6,7-dimethoxy-3-(4-methoxyphenyl)-4H-chromen-4-one **(4)** (yield: 22%).

¹H NMR (400 MHz, CDCl₃) δ: 7.44 - 7.34 (m, 7H), 7.15 - 7.05 (m, 4H), 6.97 - 6.92 (m, 2H), 6.85 (s, 1H), 5.12 (s, 2H), 3.96 (s, 3H), 3.90 (s, 3H), 3.78 (s, 3H), 3.50 (s, 3H).

### Step 4

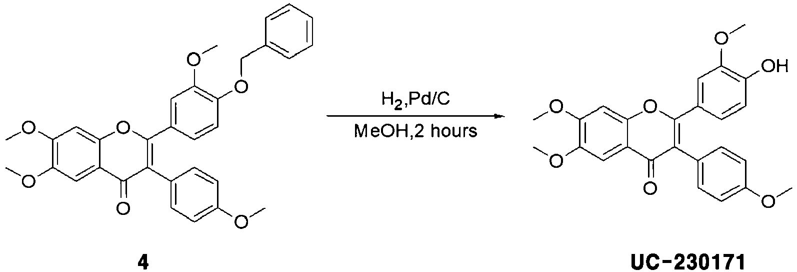

Palladium on carbon (Pd/C) (80 mg) was added to the solution of 2-(4-(benzyloxy)-3-methoxyphenyl)-6,7-dimethoxy-3-(4-methoxyphenyl)-4H-chromen-4-one (800 mg, 1.3 mmol) in EA (50 mL) and stirred under a hydrogen condition at a pressure of 60 Psi for 2 hours. Next, the solution was filtered, and the filtrate was concentrated. Subsequently, the residue was titrated with methanol to obtain 525 mg of a white solid as novel flavone derivative II **(Achem II),** 2-(4-hydroxy-3-methoxyphenyl)-6,7-dimethoxy-3-(4-methoxyphenyl)-4H-chromen-4-one **(UC-230171).**

The results of nuclear magnetic resonance spectroscopy and high-resolution mass spectrometry performed on novel flavone derivative II, the finally obtained material as described above, are as follows.
LC-MS (ESI): m/z 435.0 [M+H]⁺

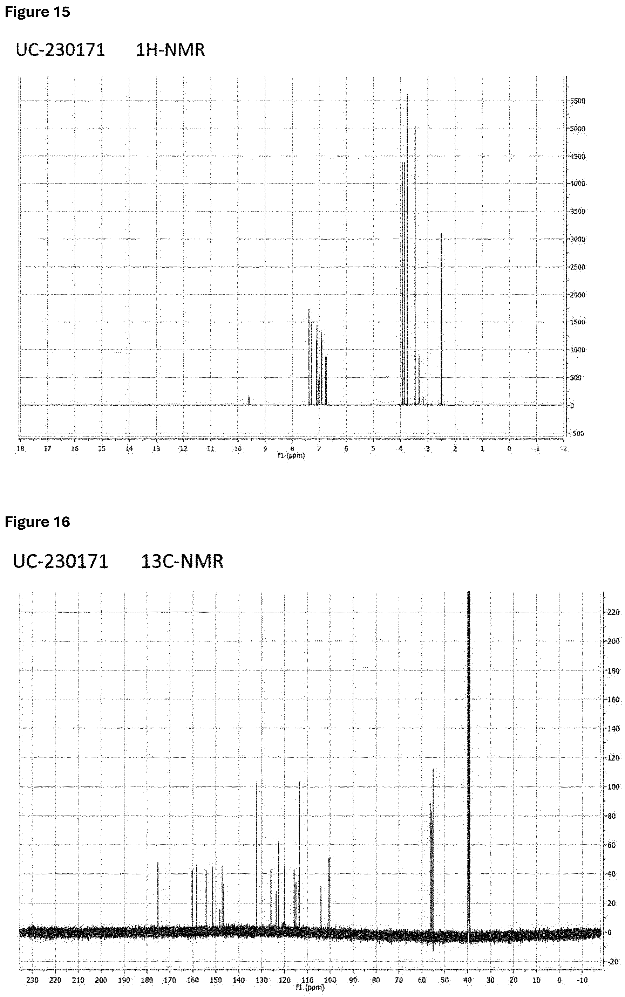

FIGS. 15 and 16 show the ¹H NMR (DMSO-d6) and ¹³C NMR (DMSO-d6) results, respectively.

### 2. Pulmonary fibrosis improvement activity test of novel flavone derivative II

### 2.1 Evaluation of cytotoxicity

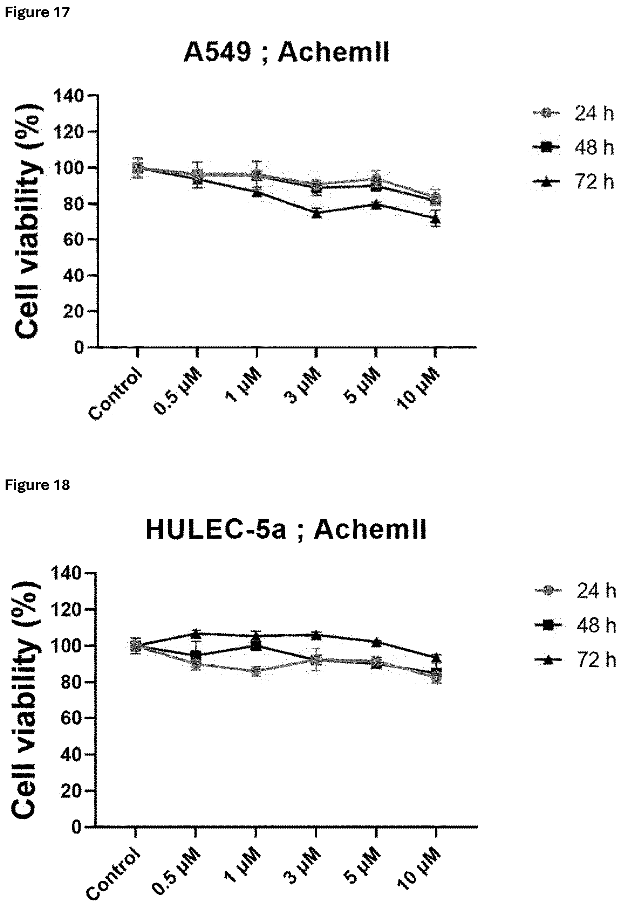

Cytotoxicity tests of novel flavone derivative II **(Achem II)** on A549 cells, HULEC-5a cells, and HLF cells were performed in the same manner as in Example 1 above, using the MTT assay. The results thereof are shown in FIGS. 17 to 19 as cell viability (%) after 24, 48, and 72 hours compared to that of the control group, the untreated sample group.

Referring to FIG. 17, the A549 cells showed a cell viability of about 80% or higher at all treatment concentrations when incubated for 24 and 48 hours while showing a cell viability of about 80% or lower at concentrations of 3 µM or higher when incubated for 72 hours.

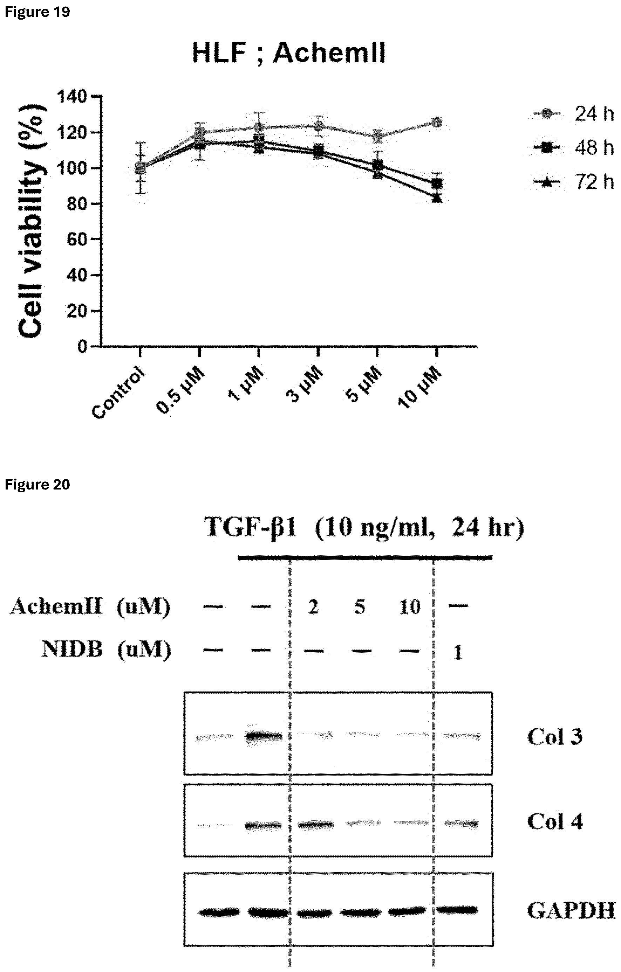

Additionally, referring to FIGS. 18 and 19, the HULEC-5a cells and the HLF cells showed a cell viability of 80% or higher at all treatment concentrations up to 10 µM when incubated for 24, 48, and 72 hours.

The incubation time and sample treatment concentration in subsequent tests took into account the cytotoxicity test results.

### 2. Effect upon TGF-β1 treatment in A549 cells

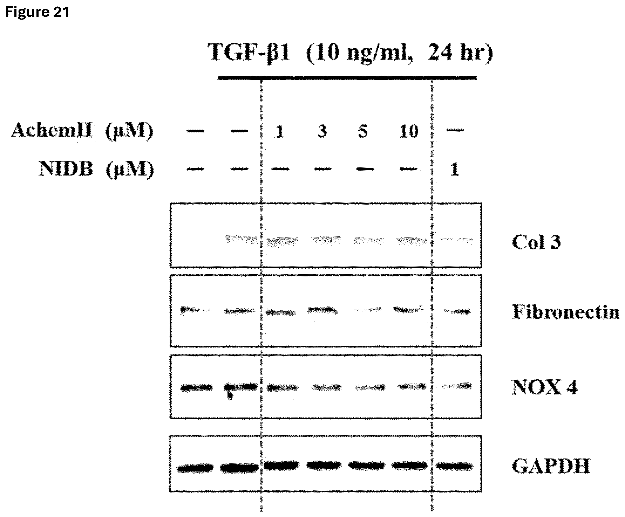

The effect of novel flavone derivative II upon TGF-β1 treatment in A549 cells was evaluated in the same manner as in Example 1 above. FIGS. 20 and 21 show the results thereof.

Referring to FIGS. 20 and 21, when treating the A549 cells with 10 ng/mL of TGF-β1 and novel flavone derivative II for 24 hours, the results thereof showed a tendency that the levels of Col3, Col4, fibronectin, and NOX4 proteins, increased by TGF-β treatment, were decreased by novel flavone derivative II.

### 3. Effect upon TGF-β1 treatment in Hulec-5a cells

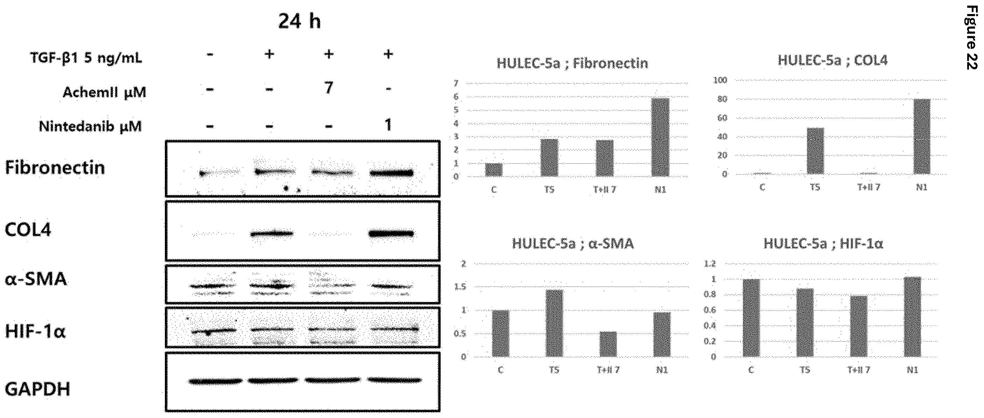

The effect of novel flavone derivative II upon TGF-β1 treatment in Hulec-5a cells was evaluated in the same manner as in Example 1 above. FIG. 22 shows the results thereof.

Referring to FIG. 22, when treated with novel flavone derivative II, the results thereof showed a tendency that the levels of fibronectin, COL4, α-SMA, and HIF-1α, increased by TGF-β1 treatment, were decreased.

### 4. Effect upon TGF-β1 treatment in HLF cells

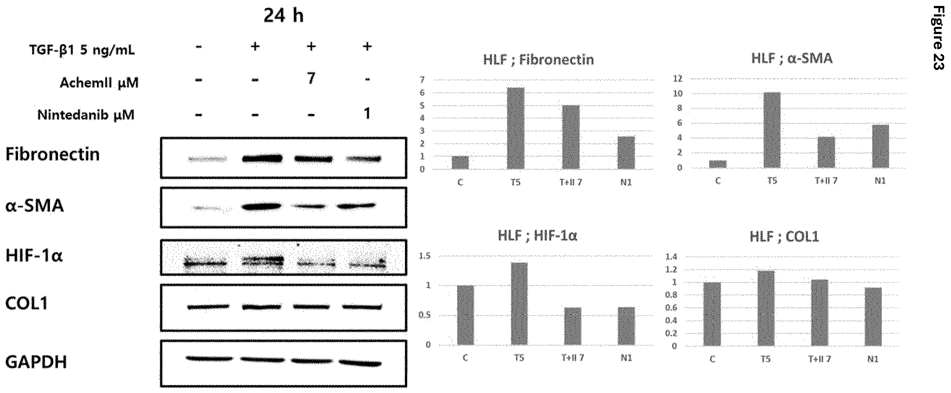

The effect of novel flavone derivative II upon TGF-β1 treatment in HLF cells was evaluated in the same manner as in Example 1 above. FIG. 23 shows the results thereof.

Referring to FIG. 23, when treated with novel flavone derivative II, the results thereof showed a tendency that the levels of fibronectin, α-SMA, HIF-1α, and COL1, increased by TGF-β1 treatment, were decreased.

### 5. Effect upon EGF treatment in A549 cells

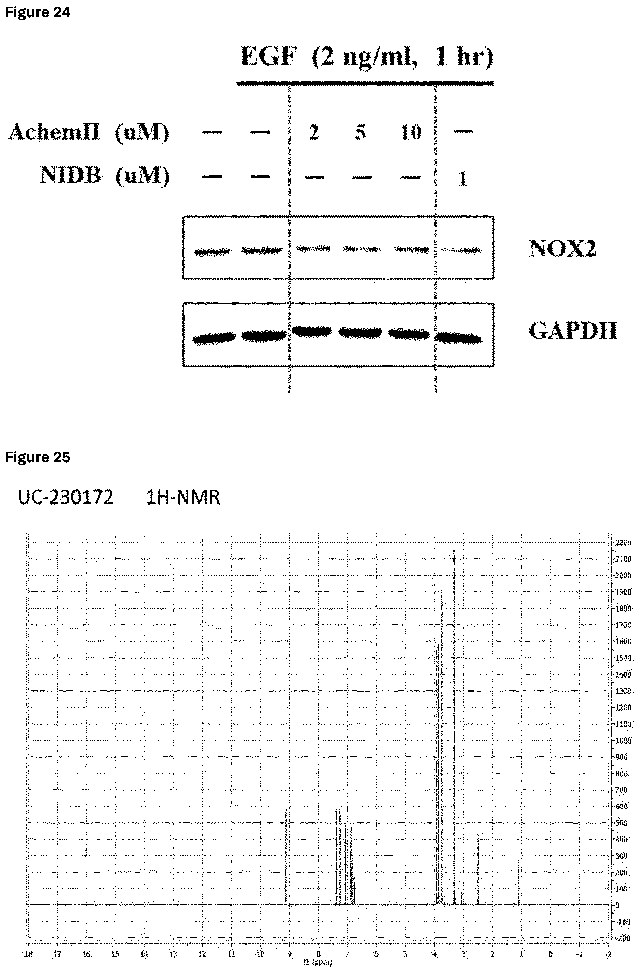

The effect of novel flavone derivative II upon EGF treatment in A549 cells was evaluated in the same manner as in Example 1 above. FIG. 24 shows the results thereof.

When treating the A549 cells with 2 ng/mL of EGF and novel flavone derivative II for 1 hour, the results thereof showed a tendency that the level of NOX2, a fibrosis-related factor increased by EGF treatment, was decreased by novel flavone derivative II.

### <Example 3> Preparation, identification, and activity test of novel flavone derivative III

### 1. Preparation of novel flavone derivative III

### Step 1

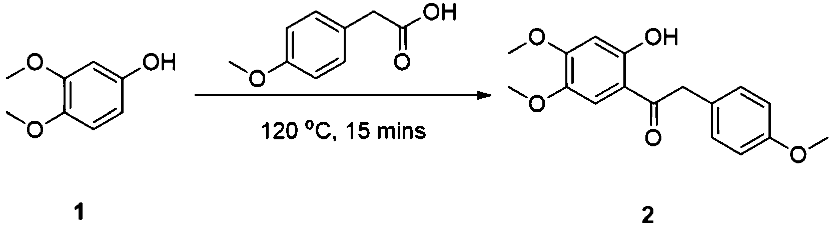

First, 2-(4-methoxyphenyl)acetic acid (CAS No: 104-01-8) (5.4 g, 32.47 mmol) was added to a solution of 3,4-dimethoxyphenol **(1)** (5.0 g, 32.47 mmol) in BF₃.Et₂O (10 mL) at room temperature under a nitrogen condition. The reaction mixture was heated to 120°C and stirred for 15 minutes. The mixture was cooled to room temperature, poured into 30 mL of water, and extracted with 30 mL of EA three times. The organic phases were combined, washed with brine, and then dried with sodium sulfate. After filtration, the solution was concentrated under vacuum to obtain 13.2 g of crude oil, which was purified through silica column (PE/EA = 10% to 50%) to obtain 4.2 g of a white solid, 1-(2-hydroxy-4,5-dimethoxyphenyl)-2-(4-methoxyphenyl)ethenone **(2)** (yield: 42.9%).

LC-MS (M+H⁺) m/z 303.3.

### Step 2

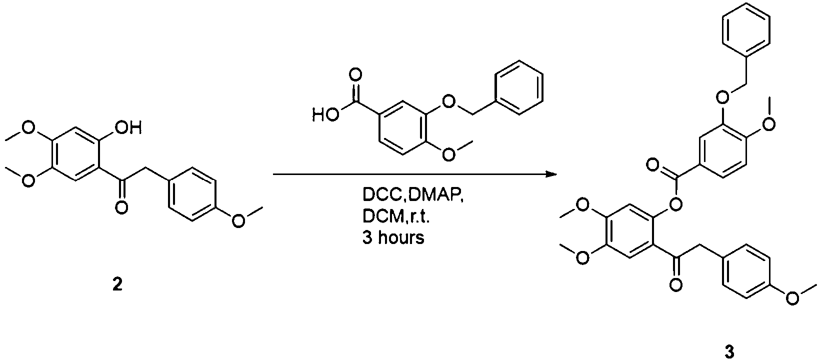

N,N'-dicyclohexylcarbodiimide (DCC) (5.5 g, 26.4 mmol) and dimethylaminopyridine(DMAP) (0.32 g, 2.64 mmol) were added to the solution of 1-(2-hydroxy-4,5-dimethoxyphenyl)-2-(4-methoxyphenyl)ethenone (2) (4.0 g, 13.24 mmol) and 3-(benzyloxy)-4-methoxybenzoic acid (4.1 g, 15.84 mmol) (CAS No.: 58452-00-9) in dichloromethane (DCM) (1000 mL). Then, the mixture was stirred at room temperature for 3 hours and concentrated under vacuum to obtain a white solid. The resulting solid was purified through silica column (PE/EA = 10% to 30%) to obtain a target compound, which was 4.8 g of a white solid, 4,5-dimethoxy-2-(2-(4-methoxyphenyl)acetyl)phenyl 3-(benzyloxy)-4-methoxybenzoate (3) (yield: 66.8%).
LC-MS (M+H⁺) m/z 543.4

### Step 3

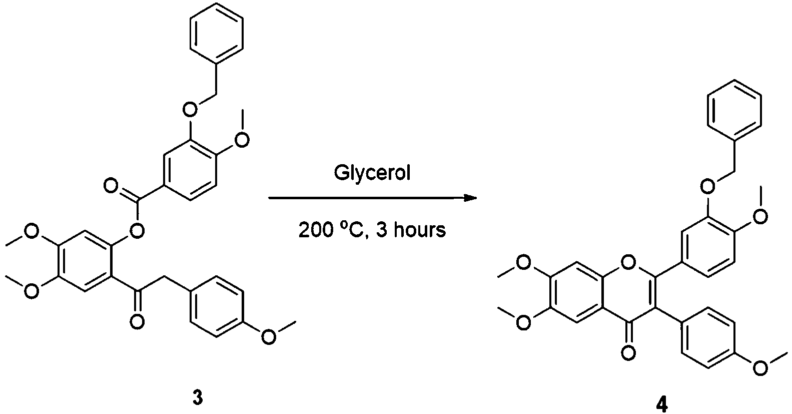

A mixture of 4,5-dimethoxy-2-(2-(4-methoxyphenyl) acetyl) phenyl 3-(benzyloxy)-4-methoxybenzoate **(3)** (4.8 g, 8.86 mmol) and glycerol (50 mL) was degassed with nitrogen three times and heated at 200°C for 3 hours. The resulting solution was cooled to room temperature, poured into 100 mL of water, and extracted with 50 mL of EA three times. The organic phases were combined, washed with brine, dried over sodium sulfate, and concentrated to obtain a crude material, which was purified through silica column to obtain 1.3 g of a white solid, 2-(3-(benzyloxy)-4-methoxyphenyl)-6,7-dimethoxy-3-(4-methoxyphenyl)-4H-chromen-4-one **(4)** (yield: 28%).
LC-MS (M+H⁺) m/z 525.4

### Step 4

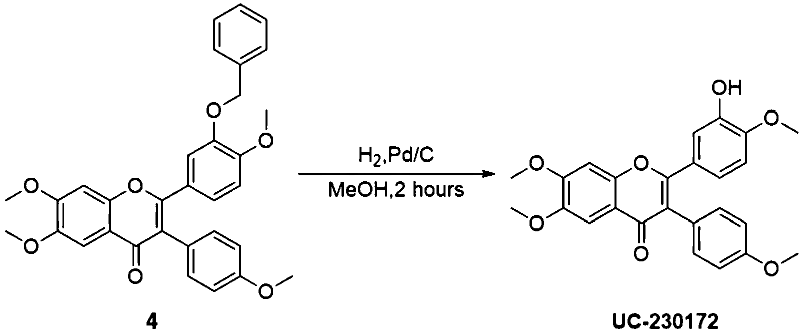

Palladium on carbon (Pd/C) (150 mg) was added to the solution of 2-(3-(benzyloxy)-4-methoxyphenyl)-6,7-dimethoxy-3-(4-methoxyphenyl)-4H-chromen-4-one **(4)** (1.3 g, 2.48 mmol) in EA (50 mL). Then, the mixture was stirred under a hydrogen condition at a pressure of 60 Psi for 2 hours. Next, the mixture was filtered, and the filtrate was concentrated. Subsequently, the resulting residue was purified by chromatography on silica gel column (PE/EA = 10% to 70%) to obtain 510 mg of a white solid as novel flavone derivative III **(Achem III),** 2-(4-hydroxy-3-methoxyphenyl)-6,7-dimethoxy-3-(4-methoxyphenyl)-4H-chromen-4-one **(UC-230172).**
LC-MS (M+H⁺) m/z 435.1

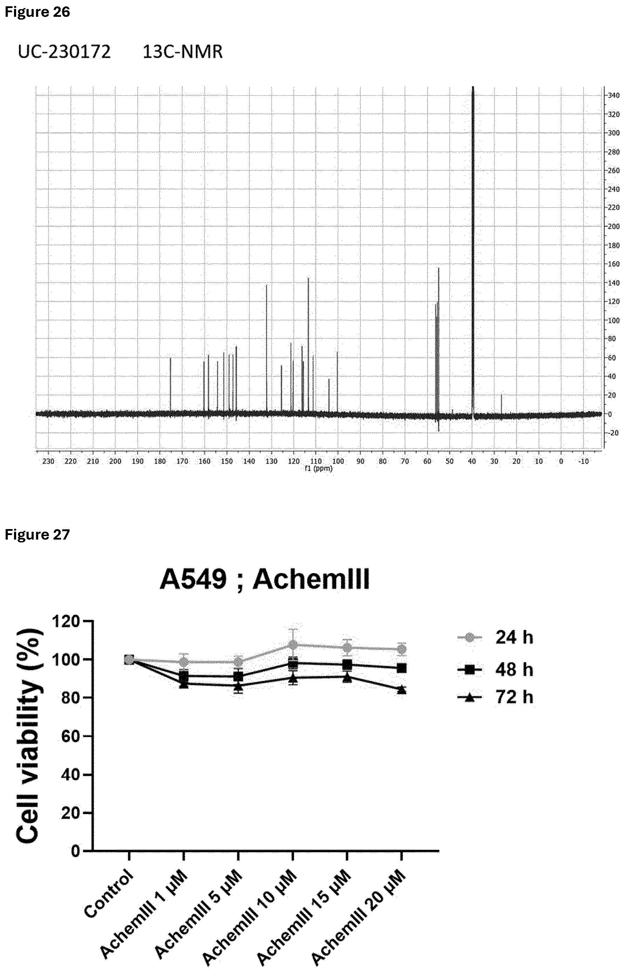

FIGS. 25 and 26 show the ¹H NMR (DMSO-d6) and ¹³ C NMR (DMSO-d6) results of novel flavone derivative III, respectively.

### 2. Pulmonary fibrosis improvement activity test of novel flavone derivative III

### 1. Evaluation of cytotoxicity

Cytotoxicity tests of novel flavone derivative III **(Achem III)** on A549 cells, HULEC-5a cells, and HLF cells were performed in the same manner as in Example 1 above, using the MTT assay. The results thereof are shown in FIGS. 27 to 29 as cell viability (%) after 24, 48, and 72 hours compared to that of the control group, the untreated sample group.

Referring to FIG. 27, the A549 cells showed a cell viability of 80% or higher at all treatment concentrations up to 20 µM when incubated for 24, 48, and 72 hours.

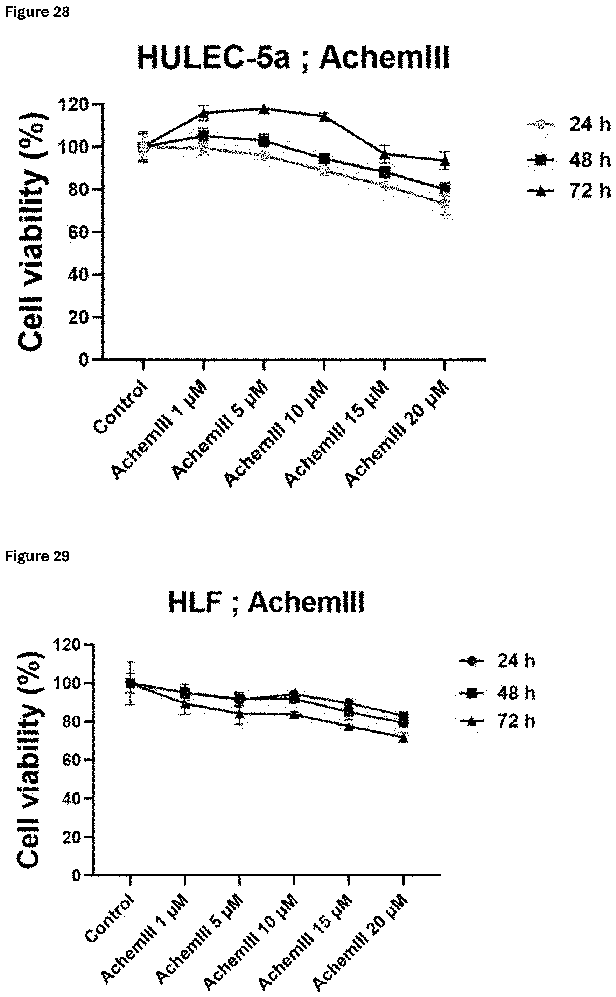

Additionally, referring to FIG. 28, the HULEC-5a cells showed a cell viability of 80% or higher at concentrations up to 15 µM when incubated for 24, 48, and 72 hours while showing a cell viability of 80% or lower at a concentration of 20 µM when incubated for 24 hours.

Furthermore, referring to FIG. 29, the HLF cells showed a cell viability of 80% or higher at all treatment concentrations up to 10 µM when incubated for 24, 48, and 72 hours while showing a cell viability of 80% or lower at concentrations of 15 and 20 µM when incubated for 24 hours.

The incubation time and sample treatment concentration in subsequent tests took into account the cytotoxicity test results.

### 2. Effect upon TGF-β1 treatment in A549 cells

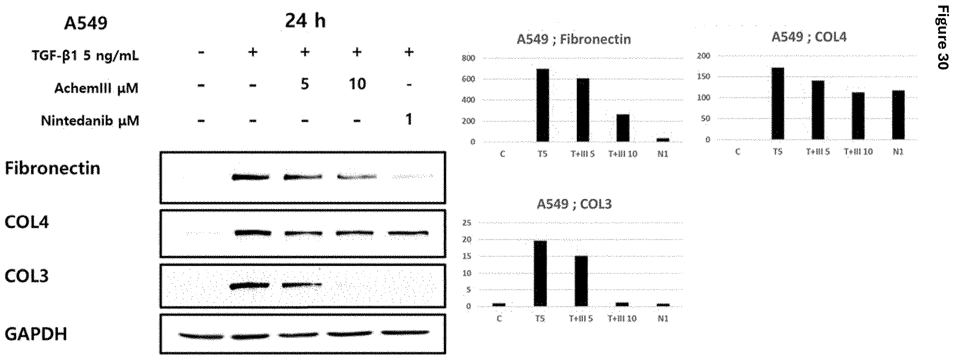

The effect of novel flavone derivative III upon TGF-β1 treatment in A549 cells was evaluated in the same manner as in Example 1 above. FIG. 30 shows the results thereof.

Referring to FIG. 30, when treating the A549 cells with 5 ng/mL of TGF-β1 and novel flavone derivative III for 24 hours, the results thereof showed a tendency that the levels of fibronectin, COL4, and COL3, increased by TGF-β1 treatment, were decreased by novel flavone derivative III.

### 3. Effect upon TGF-β1 treatment in Hulec-5a cells

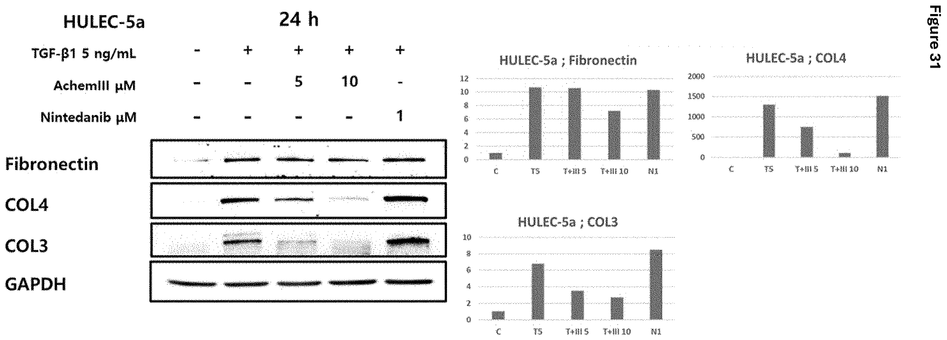

The effect of novel flavone derivative III upon TGF-β1 treatment in Hulec-5a cells was evaluated in the same manner as in Example 1 above. FIG. 31 shows the results thereof.

Referring to FIG. 31, when treating the Hulec-5a cells with 5 ng/mL of TGF-β1 and novel flavone derivative III for 24 hours, the results thereof showed a tendency that the levels of fibronectin, COL4, and COL3, increased by TGF-β1 treatment, were decreased.

### 4. Effect upon TGF-β1 treatment in HLF cells

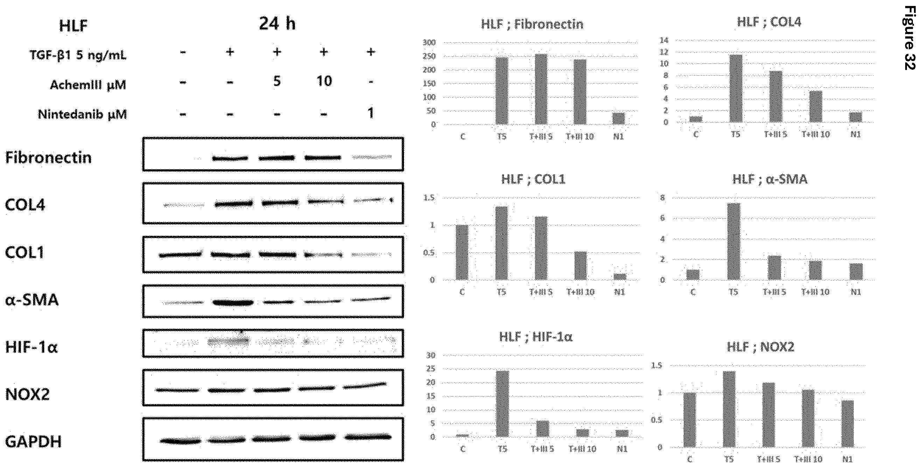

The effect of novel flavone derivative III upon TGF-β1 treatment in HLF cells was evaluated in the same manner as in Example 1 above. FIG. 32 shows the results thereof.

Referring to FIG. 32, when treating the HLF cells with 5 ng/mL of TGF-β1 and novel flavone derivative III for 24 hours, the results thereof showed a tendency that the levels of fibronectin, COL4, COL1, α-SMA, HIF-1α, and NOX2, increased by TGF-β1 treatment, were decreased.

## Claims

1. A compound being a flavone derivative of Formula 4, a hydrate thereof, or a solvate thereof, 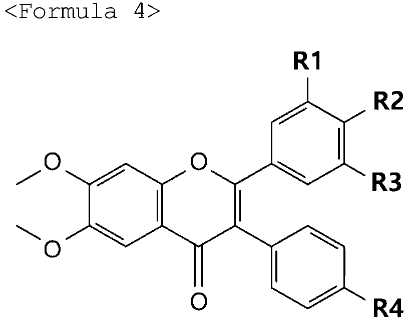 wherein in Formula 4, R1, R2, R3, and R4 are each independently hydrogen (H), a hydroxy group (-OH), a methyl group (-CH₃), or a methoxy group (-OCH₃).

2. The compound of claim 1, wherein R1 is hydrogen or the hydroxy group,
R2 is the hydroxy group or the methoxy group,
R3 is hydrogen, the methoxy group, or the hydroxy group, and
R4 is the methoxy group.

3. The compound of claim 1, wherein the flavone derivative is a compound of Formula 1, 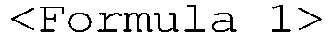 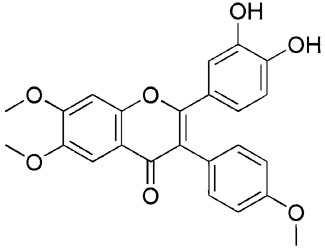 **2-(3,4-dihydroxyphenyl)-6,7-dimethoxy-3-(4-methoxyphenyl)-4H-chromen-4-one**

4. The compound of claim 1, wherein the flavone derivative is a compound of Formula 2, 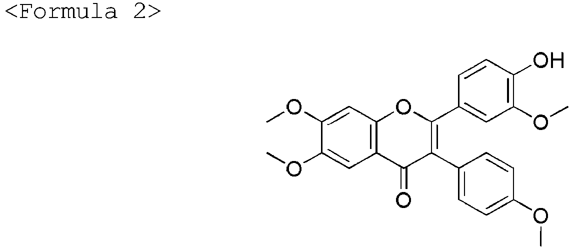 **2-(4-hydroxy-5-methoxyphenyl)-6,7-dimethoxy-3-(4-methoxyphenyl)-4H-chromen-4-one**

5. The compound of claim 1, wherein the flavone derivative is a compound of Formula 3, 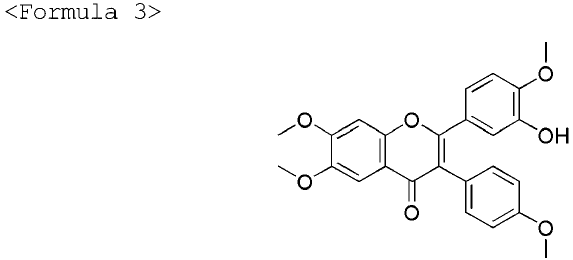 2-(4-methoxy-5-hydroxyphenyl)-6,7-dimethoxy-3-(4-methoxyphenyl)-4H-chrome n-4-one

6. A composition for improving pulmonary fibrosis, the composition comprising a flavone derivative of Formula 4, a hydrate thereof, or a solvate thereof as an active ingredient, 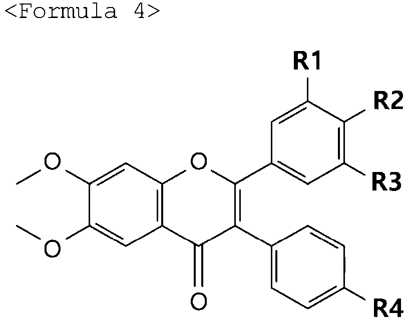 wherein in Formula 4, R1, R2, R3, and R4 are each independently hydrogen (H), a hydroxy group (-OH), a methyl group (-CH₃), or a methoxy group (-OCH₃).

7. The composition of claim 6, wherein R1 is hydrogen or the hydroxy group,
R2 is the hydroxy group or the methoxy group,
R3 is hydrogen, the methoxy group, or the hydroxy group, and
R4 is the methoxy group.

8. The compound of claim 6, wherein the flavone derivative is a compound of Formula 1, 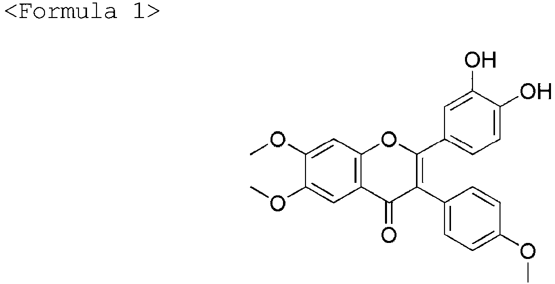 2-(3,4-dihydroxyphenyl)-6,7-dimethoxy-3-(4-methoxyphenyl)-4H-chromen-4-one

9. The compound of claim 6, wherein the flavone derivative is a compound of Formula 2, 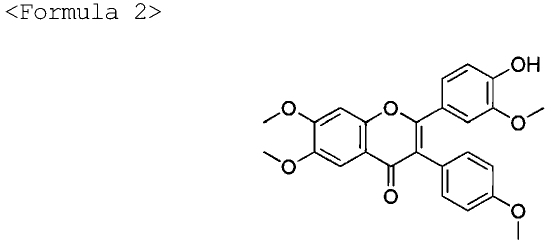 **2-(4-hydroxy-5-methoxyphenyl)-6,7-dimethoxy-3-(4-methoxyphenyl)-4H-chromen-4-one**.

10. The compound of claim 6, wherein the flavone derivative is a compound of Formula 3, 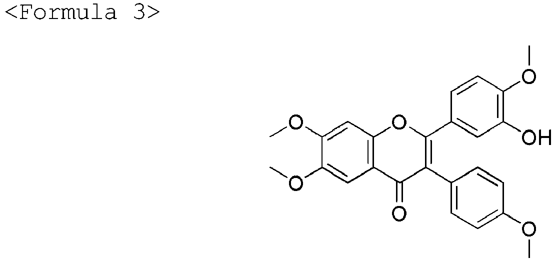 **2-(4-methoxy-5-hydroxyphenyl)-6,7-di**methoxy-3-(4-meth**oxyphenyl)-4H-chromen-4-one**

11. The composition of 6, wherein the pulmonary fibrosis is idiopathic pulmonary fibrosis.

12. The composition of claim 6, wherein the composition is a pharmaceutical composition.

13. The composition of claim 6, wherein the composition is a food composition.
